# EUROPEAN PATENT SPECIFICATION

(11) **EP 3 049 103 B1**
(45) Date of publication and mention of the grant of the patent: **24.06.2020**
(21) Application number: 14848904.0
(22) Date of filing: 24.09.2014
(51) Int. Cl.: A61K 39/00, C07F 9/09

(54) **COMPOSITIONS AND METHODS FOR REDUCING ANTIGEN-SPECIFIC IMMUNOGENICITY**
ZUSAMMENSETZUNGEN UND VERFAHREN ZUR VERMINDERUNG EINER ANTIGENSPEZIFISCHEN IMMUNOGENITÄT
COMPOSITIONS ET MÉTHODES POUR RÉDUIRE L'IMMUNOGÉNICITÉ SPÉCIFIQUE D'ANTIGÈNE

(30) Priority: 24.09.2013 US 201361881857 P
(43) Date of publication of application: 03.08.2016
(73) Proprietor: The Research Foundation for the State University of New York, Amherst, NY 14228-2567 (US)
(72) Inventor: BALU-IYER, Sathy, Amherst, NY 14228 (US); KOSLOSKI, Matthew P., Wilmette, IL 60091 (US); SHETTY, Krithika Arun, Buffalo, NY 14214 (US); SCHNEIDER, Jennifer, Leigh, Buffalo, NY 14201 (US); FATHALLAH, Anas, San Diego, CA 92101 (US)
(74) Representative: Mathys & Squire
(86) International application number: PCT/US2014/057234
(87) International publication number: WO 2015/048145

(56) References cited:
- ANAS M FATHALLAH ET AL: "IMMUNOGENICITY AND PHARMACOKINETICS OF SUBCUTANEOUSLY ADMINISTERED LARGE PROTEINS", MID-ATLANTIC GRADUATE STUDENT SYMPOSIUM IN MEDICINAL CHEMISTRY, PHARMACOGNOSY, AND PHARMACEUTICAL SCIENCE (MAGSS 2013) | OHIO STATE UNIVERSITY, COLUMBUS, OHIO | JUNE 9 -11,2013, OHIO STATE UNIVERSITY, , vol. 46th 9 June 2013 (2013-06-09), page 20, XP008183456, Retrieved from the Internet: URL:http://www.pharmacy.ohio-state.edu/for ms/currentstudents/magss2013/2013_MAGSS_Fi nal_Program-and-abstracts.pdf [retrieved on 2017-04-27]
- MICLEA RAZVAN D ET AL: "O-phospho-L-serine, multi-functional excipient for B domain deleted recombinant factor VIII", THE AAPS JOURNAL, SPRINGER-VERLAG, NEW YORK, vol. 9, no. 2, 29 June 2007 (2007-06-29), pages E251-E259, XP035718900, DOI: 10.1208/AAPSJ0902028
- PUNEET GAITONDE ET AL: "Intravenous administration of Factor VIII-O-Phospho-L-Serine (OPLS) complex reduces immunogenicity and preserves pharmacokinetics of the therapeutic protein", EUROPEAN JOURNAL OF PHARMACEUTICAL SCIENCES., vol. 66, 1 January 2015 (2015-01-01), pages 157-162, XP055368062, NL ISSN: 0928-0987, DOI: 10.1016/j.ejps.2014.10.010
- FATHALLAH ET AL.: 'IMMUNOGENICITY AND PHARMACOKINETICS OF SUBCUTANEOUSLY ADMINISTERED LARGE PROTEINS.' 2013 MID-ATLANTIC GRADUATE STUDENT SYMPOSIUM (MAGSS) AT THE OHIO STATE UNIVERSITY 09 June 2013, page 20, XP008183456
- PUROHIT ET AL.: 'Lower Inhibitor Development in Hemophilia A Mice following Administration of Recombinant Factor VIII-O-Phospho-L-serine Complex';' J. BIO. CHEM. vol. 280, 2005, pages 17593 - 17600, XP055334532
- FATHALLAH ET AL.: 'Immunogenicity of Subcutaneously Administered Therapeutic Proteins-a Mechanistic Perspective';' THE AAPS JOURNAL vol. 15, no. 4, 16 July 2013, pages 897 - 900, XP055334533

## Description

### STATEMENT REGARDING FEDERALLY SPONSORED RESEARCH

This invention was made with government support under contract HL-70227 awarded by the National Institutes of Health. The government has certain rights in this invention.

### FIELD OF THE DISCLOSURE

This disclosure relates generally to the field of immunogenicity of antigenic proteins. More particularly, this disclosure relates to compositions and methods for: (i) reducing the immunogenicity, (ii) improving the pharmacokinetics, and (iii) induction of tolerance, to biotherapeutics, including therapeutic proteins, peptides and antibodies.

### BACKGROUND OF THE DISCLOSURE

Biotherapeutics, which includes recombinant proteins, peptides and antibodies, represent a growing class of pharmaceutical agents. This class is already being used to treat a broad range of diseases. While treatments using these agents can be highly effective, such treatments often encounters problems related to stability and immunogenicity. It is not uncommon for therapeutic proteins to form aggregates, wherein unfolded therapeutic proteins associate with each other and form complexes that can lead to undesired immune responses. Equally, if not more, concerning is the inherent immunogenicity of such therapies which often leads to antibody-based immune responses that impact their safety and efficacy. For example, antibody-based immune responses (e.g., anti-drug antibodies) can not only alter pharmacokinetics (e.g. increase the rate of clearance/reduced circulation time) but also abrogate the pharmacological activity of the protein. The latter occurs when the antibody-based immune response involves the development of neutralizing antibodies. Over time, as the titers of neutralizing antibodies increase, the therapy becomes less effective and can lead to additional morbidities and sometimes even mortality, especially in cases where no alternative therapies exist.

The "immunogenicity" of a biotherapeutic is influenced by various factors, including route of administration, concomitant medications, drug doses and treatment scheduled, genetics, age, gender, immune and nutritional status, disease characteristics, the size and structure of the drug, how "human" the drug is, the clearance rate of immune complexes, and drug solubility. Because there are so many factors which induce immunogenicity, there can be a broad range of "immunogenicities" for a particular biotherapeutic between different studies.

Numerous examples exist for the development of anti-drug antibodies against biotherapeutics. Many examples involve monoclonal antibodies, which can include chimeric, humanized and even fully human antibodies. A specific example is biologic anti-tumor necrosis factor (anti-TNF) therapies such as adalimumab (Humira®) and infliximab (Remicade®), which are used to treat autoimmune disease. The formation of anti-drug antibodies is recognized as the mechanism explaining the failure of anti-TNF biotherapeutics in the treatment of Rheumatoid Arthritis ("RA"). Anti-drug antibodies developed towards anti-TNF therapeutics reduce the clinical effectiveness of these therapeutics and necessitate higher dosing or discontinuation. Neutralizing antibodies cause 12% to 44% of RA patients to develop resistance or adverse reactions to anti-TNF agents. Because of antidrug antibody formation, at least 1/3 of RA patients do not respond to treatment or lose initial responsiveness. Each of the currently marketed anti-TNF biotherapeutics (Remicade® (infliximab), Humira® (adalimumab), Simponi® (golimumab), Enbrel® (etanercept), and Cimzia® (PEGylated certolizumab)) has a respective range of "immunogenicity."

Other examples involve protein-based replacement therapies, including but not limited to replacement therapies (e.g. Factor VIII) for coagulation disorders such as hemophilia and enzyme replacement therapies for lysosomal storage diseases such as Pompe disease. In a specific example, patients suffering from Pompe disease possess a deficiency in the enzyme acid alpha glucosidase ("GAA"). The current treatment for the disease is enzyme replacement therapy with alglucosidase alfa, which is marketed as Myozyme® and Lumizyme®. Like the anti-TNF biotherapeutics mentioned above, these drugs are immunogenic, often resulting in undesirable immune responses that ultimately render the therapy less-effective or even ineffective.

Several approaches have been used and/or investigated to minimize immunogenicity of biotherapeutics, including but not limited to development of less-immunogenic formulations, modifying treatment regimens, use of steroids (i.e. generalized immunosuppression), and various delivery approaches. Each approach has its own set of drawbacks and limitations.

In addition to immunogenicity, another concern facing subcutaneous routes of administration of biotherapeutics is their incomplete bioavailability, which is presumably due, in part, to the degradation of the biotherapeutic at or near the site of injection.

Therefore, a safe and effective approach to minimize immunogenicity, improve pharmacokinetics, and induce antigen-specific immune tolerance is desirable.

Anas M. Fathallah et al: "Immunogenicity and pharmacokinetics of subcutaneously administered large proteins", mid-atlantic graduate student symposium in medicinal chemistry, Pharmacognosy and pharmaceutical science (MAGSS 2013), Ohio State University, Columbus, Ohio, June 9 - 11, 2013, Ohio State University discloses that administration of large proteins via the sc route with modulatory agents such as OPLS can reduce immunogenicity.

Miclea Razvan D et al: "O-phospho-L-serine, multi-functional excipient for B domain deleted recombinant factor VIII", The AAPS Journal, Springer-Verlag, New York, vol. 9, no. 2, 1 January 1901 (1901-01-01), pages E251-E259 discloses that specific molecular interaction of BDDrFVIII occurs with OPLS resulting in less protein aggregation and less immunogenicity.

Puneet Gaitonde et al: "Intravenous administration of Factor VIII-O-Phospho-L-serine (OPLS) complex reduces immunogenicity and preserves pharmacokinetics of the therapeutic protein", European Journal of pharmaceutical sciences., vol. 66, 1 January 2015 (2015-01-01), pages 157 to 162 investigates FVIII immune response and pharmacokinetics on administration upon intravenous administration of FVIII-OPLS complex.

Purohit et al: "Lower inhibitor development in Hemophilia A Mice following administration of Recombinant Factor VIII-O-Phospho-L-serine Complex", J. BIO. CHEM, vol. 280, 2005, pages 17593 - 17600 investigates the hypothesis that complexation of O-phospho-1-serine, the head group of phosphatidylserine, with the C2 domain can reduce the overall immunogenicity of factor VIII.

Fathallah et al.: "Immunogenicity of subcutaneously administered therapeutic proteins a mechanistic perspective", The AAPS Journal, vol. 15, no. 4, 16 July 2013, pages 897 to 900 discloses a mechanistic perspective of immunogenicity of therapeutic proteins administered via the sc route and discuss strategies and opportunities for novel therapeutic approaches to mitigate immunogenicity.

### SUMMARY OF THE DISCLOSURE

The invention is set out in the appended set of claims. The embodiments and/or examples of the following description which are not covered by the appended claims are not considered to be part of the present invention. According to aspects of the invention, there are provided compositions for use in a method of inducing immune tolerance according to the appended claims.

The present disclosure describes methods and compositions for reducing the immunogenicity of antigens (e.g. biotherapeutics), improving their pharmacokinetic properties, and inducing specific immunological tolerance to such antigens.

This present disclosure is based on the surprising observation that concomitant or sequential administration of certain lipidic particles or OPLS, and antigens, even in the absence of association of the antigen with OPLS or incorporation of the antigen into lipidic particles (and/or shielding of immunogenic epitopes) (i.e. formation of complexes), results in both immunological hypo-responsiveness against the antigen and an improvement in its pharmacokinetic properties. This is especially surprising considering that the reduction in immunogenicity is seen even when administration takes place via the subcutaneous route, which is widely believed to be highly immunogenic due to the presence of high numbers of dendritic cells (i.e. high immunologic exposure).

Immunological tolerance can be viewed as a continuum that ranges from immunological hypo-responsiveness to non-responsiveness to an antigen. For therapeutic purposes, it is generally preferred that immunological tolerance is specific to the antigen in question so as to avoid complications that arise from generalized immunosuppression. In the context of this disclosure "immunological tolerance" to an antigen is evidenced by any one or combination of the following: (a) increases in one or more of the following: (i) T regulatory cells, (ii) TGF-b, and/or (iii) IL-10, and/or (b) decreases in one or more of the following: (i) antigen-specific antibody titers, (ii) B cells, including antigen-specific memory B cells, (iii) IL-6, (iv) IL-17, (v) CD40, (vi) CD80, and/or (vii) CD86, and/or (c) hypo-responsiveness following re-challenge with the antigen. One or more of these identifiers can be evaluated in culture conditions. By "specific" it is meant that an immune response to non-relevant antigens (antigens that were not complexed to or co-administered, either concomitantly or sequentially, with the lipidic compositions of the present disclosure) is not affected.

In one aspect, this disclosure provides a method for inducing immune tolerance to agents (such as therapeutic agents). The agents are administered in conjunction with the administration of OPLS. In one embodiment, the agent and OPLS are administered as separate compositions. The administrations may be carried out at the same time (concomitantly) or may be carried out one after the other (sequentially). In one embodiment, the OPLS and the agent may be combined before administration, but the association of the OPLS and the agent is low or absent (i.e. undetectable).

In one embodiment, the bioavailability of the biotherapeutics is increased by improving lymphatic uptake after sc administration. While agents (e.g. albumin) that act as volume expanders have been explored in the past, these agents have no ability to reduce the immunogenicity of the co-administered biologic. In the present disclosure, it was observed that administration of a biologic in a hypertonic buffer could increase bioavailability as well as induce immune tolerance.

In one embodiment, the therapeutic agent is administered in conjunction with OPLS at a sub-therapeutic dose. At this dose, the therapeutic agent does not exert a clinically relevant effect for the indication for which it is intended. However, as described in this disclosure, surprisingly, it is able to induce specific immune tolerance toward the agent. The therapeutic and OPLS may be administered in the same composition or may be administered separately (concomitantly or sequentially).

In one embodiment, the antigen (such as a therapeutic agent) is administered in conjunction with OPLS (as a single formulation, or separate formulations - whether administered concomitantly or sequentially), wherein the agent is at a therapeutic dose. In one embodiment, if administered as a single formulation, the OPLS and the agent are loosely bound (K_{d} of higher than 10 µM and up and including 500 µM) or unbound (K_{d} of higher than 500 µM). In one embodiment, if administered as a single formulation, the agent and OPLS are combined immediately prior to administration so that there is no detectable complexation of the OPLS and the antigen.

In one embodiment, the administration of the agent and OPLS is done via a cutaneous route. The cutaneous route may be a sub-cutaneous injection, transdermal or intradermal delivery, topical application or any other delivery method via the skin.

In one aspect, this disclosure provides a method for inducing immune tolerance by ex vivo exposure to a therapeutic antigen and OPLS. The method comprises obtaining dendritic cells from an individual, contacting the dendritic cells in vitro with OPLS and the antigen, and returning the dendritic cells to the host.

In one aspect, the present disclosure provides kits for inducing immune tolerance. The kits comprise one or more of the following: OPLS or other serine containing lipid formulations, therapeutic agents, hypertonic buffer, administration devices and agents, instructions for administration of the various compositions.

### BRIEF DESCRIPTION OF THE FIGURES

Figure 1. Effect of OPLS on the cytokine profile of bone marrow derived dendritic cells (BMDC). TGF-β, TNF-α and IL-6 concentrations are shown as a function of OPLS concentration.
Figure 2. Binding of OPLS to biotherapeutics. Dissociation constants (K_{d}) ranges from 8.4 uM for FVIII (strong binding) to 2573 uM for OVA (essentially no binding).
Figure 3. Murine antibody titers following subcutaneous coadministration of FVIII and OPLS (0.4 mg/5 IU and 5 mg/5 IU) vs. FVIII alone (5 IU). The OPLS and FVIII were combined just prior to administration at room temperature and there were no detectable complexes of antigen and OPLS.
Figure 4. Average FVIII bioactivity (plasma) as a function of time following intravenous administration in mice.
Figure 5 (A) Plasma concentrations and (B) lymphatic concentrations following subcutaneous administration of FVIII and OPLS-FVIII in mice.
Figure 6. Murine antibody titer levels following subcutaneous administration of protein vs. subcutaneous co-administration of protein and lipid. (a) administration of ovalbumin vs. co-administration of ovalbumin and 5 mg OPLS, (b) administration of FVIII vs. co-administration of FVIII and OPLS at various concentrations, (c) administration of acid alpha glucosidase (GAA) protein vs. co-administration of GAA protein and either O-Phospho-D-Serine ("OPDS") or OPLS, (d) administration of Humira® (adalimumab) vs. co-administration of Humira® (adalimumab) and 5 mg OPLS.
Figure 7. Murine antibody titers following subcutaneous co-administration of human IgG and OPLS (0.4 mg/1ug) vs. subcutaneous administration of IgG alone. Like Figure 7, greater effects are expected at OPLS dose equal to or greater than 5 mg. Human IgG in this example is a human plasma IgG isolate.
Figure 8. Murine antibody titers following subcutaneous co-administration of rituximab (RXT) and OPLS (0.4 mg/1ug and 5 mg/1ug) vs. subcutaneous administration of rituximab alone.
Figure 9. Murine antibody titers following subcutaneous administration of acid alpha glucosidase (GAA) peptide (SEQ: YIFLGPEPKSVVQ) vs. subcutaneous co-administration of GAA peptide and either PS-liposomes (1:10,000 as described below) or 5 mg OPLS. Note that the GAA peptide is the most prominent T-cell epitope of full length GAA (Molecular Genetics and Metabolism, 2012. 106(2): p. 189-195).
Figure 10. Murine antibody titer levels following subcutaneous administration of GAA protein vs. subcutaneous co-administration of GAA protein and lipid. The lipids used were PS-liposomes (PS/PC) and PG30.

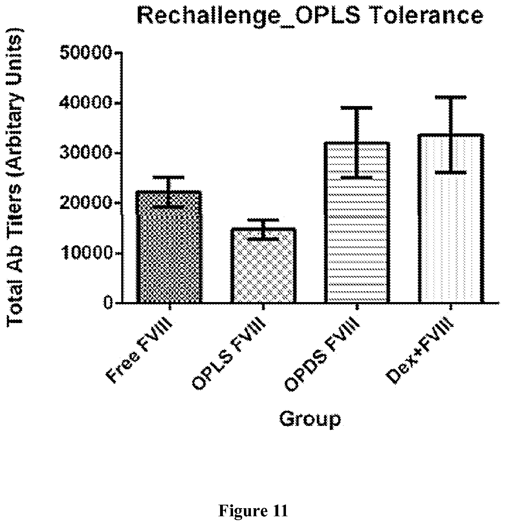

Figure 11. FVIII-specific tolerance/hypo-responsiveness using O-Phospho-L-Serine (hereinafter referred to as "OPLS"). (a) Baseline shows comparison of murine anti-FVIII antibody titers following subcutaneous co-administration of: (i) FVIII and OPLS, (ii) FVIII and O-Phospho-D-Serine (hereinafter referred to as "OPDS"), (iii) FVIII and Dexamethasone ("Dex"), compared to administration of FVIII alone. (b) Rechallenge shows comparison of murine anti-FVIII antibody titers in each group following subcutaneous rechallenge with free FVIII. Co-administration (s.c.) of FVIII with OPLS led to antigen-(FVIII-) specific hypo-responsiveness upon rechallenge whereas co-administration (s.c.) of FVIII with either OPDS or Dexamethasone, a general immunosuppressant, did not.
Figure 12. FVIII-specific tolerance/ hypo-responsiveness using PS-liposomes. (a) Murine anti-FVIII neutralizing antibody titers following reverse vaccination with various lipid compositions (phosphatidylserine (PS), phosphatidylcholine (PC), Phosphatidylglycerol (PG)) compared to free FVIII and FVIII plus dexamethasone (Dex); (b) Murine anti-Ovalbumin (Ova) titers in each group following rechallenge with Ova to show that mice respond normally to Ova following reverse vaccination procedure.
Figure 13. Anti GAA titers for GAA and GAA plus OPLS treated animals after GAA rechallenge.
Figure 14. Anti GAA titers for GAA and GAA plus OPLS treated animals after GAA rechallenge GAA tolerance data with PS-liposomes.

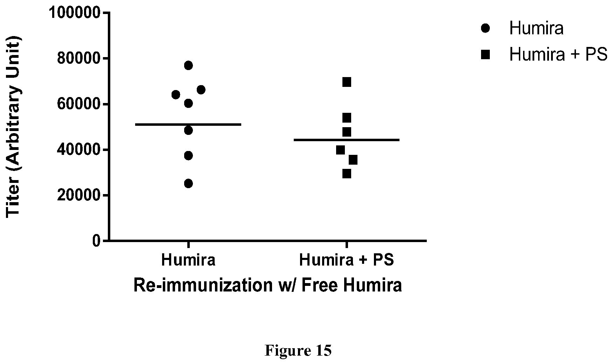

Figure 15. Humira® (adalimumab) tolerance data with PS-liposomes. Murine models were subjected to (a) free Humira and (b) Humira with phosphatidylserine (PS) liposomes. After respective exposure to (a) free Humira and (b) Humira with PS liposomes, the models were re-challenged with free Humira and immunologic titers were measured in Arbitrary Units. Those which were originally subjected to a regimen with Humira and PS liposomes produced a statistically significant reduced immunologic titer than did those which were originally subjected to only free-form Humira.
Figure 16. Administration of Factor VIII with PS liposomes induces CD4+FoxP3+ T-regulatory cell upregulation. Experimental groups were administered either (a) Free Factor VIII or (b) Factor VIII with PS liposomes. The group administered (b) Factor VIII with PS liposomes had a statistically significant relative greater upregulation of CD4+FoxP3+ T-regulatory cells than did those administered free-form Factor VIII
Figure 17. Role of Memory B cells in PS mediated hypo responsiveness. Mice that received memory B cell from a PS-FVIII treated donor mice exhibited significantly reduced anti-FVIII antibody titers.
Figure 18. Neutralizing titer data from mice which had received four weeks of tolerance induction approach wherein antigen and lipidic particles are administered distal from one another followed by four weeks of free FVIII re-challenge.
Figure 19. Total anti-FVIII titer data for mice undergoing tolerance induction approach wherein antigen and lipidic particles are administered distal from one another.

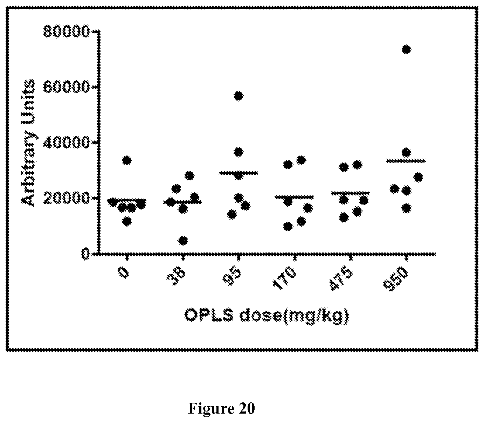

Figure 20. Anti-KLH IgG titer levels obtained from a short term, repeat dose (28 daily doses) TDAR study conducted in CD-1 mice. No statistical significance was found between mean titer levels of any OPLS treated group and the vehicle treated group. The data indicate that even at the highest dose evaluated, OPLS does not alter the state of general immune-competence of the animals.

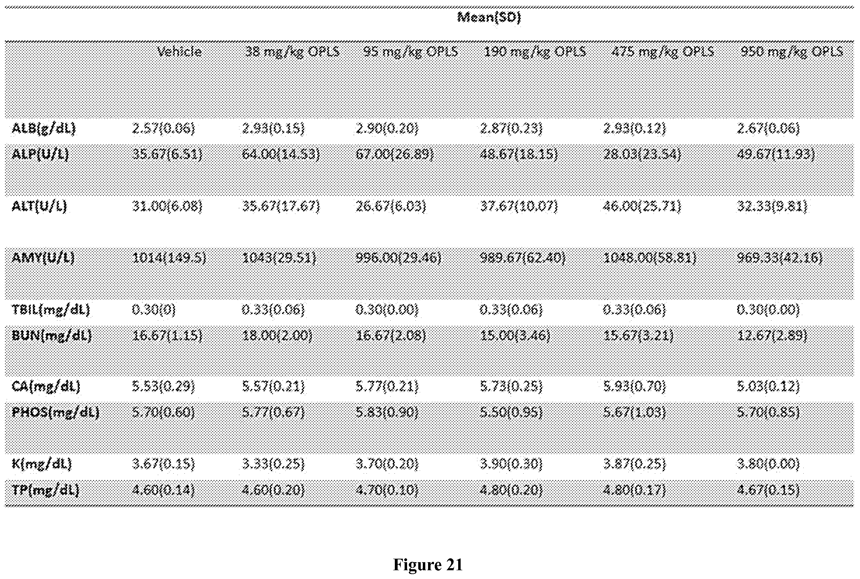

Figure 21. Effect of 28 daily injections of a range of OPLS doses on plasma chemistry markers of CD-1 mice. Samples were collected from animals 1 day after the termination of dosing. The data indicate that there is no impact of the given OPLS dose on renal and hepatic function at even the highest evaluated dose of OPLS.

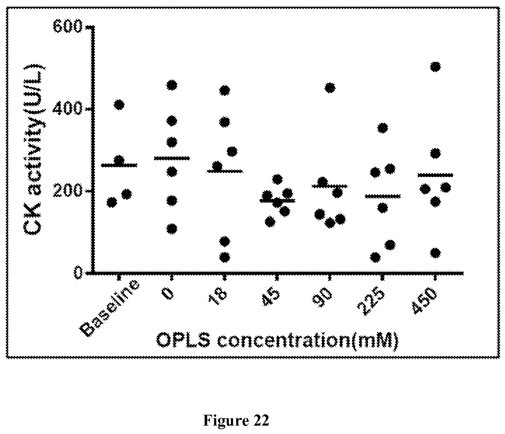

Figure 22. Plasma Creatine Kinase (CK: an enzyme with high expression in myocytes, elevation of plasma CK is an indication of damage to muscle tissue) activity levels after 28 daily s.c. injections of OPLS to CD-1 mice. No visible injection site pathology was observed during macroscopic evaluations of the injection site tissue upon necropsy in any of the OPLS treated groups. No statistical significance was found between mean plasma CK activity levels of any OPLS treated group and the vehicle treated group. The data indicate that even at the highest dose evaluated, OPLS does not cause muscular degradation at the injection site.

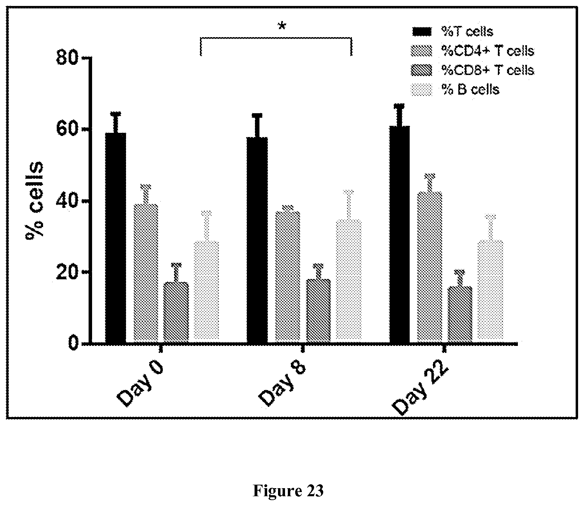

Figure 23. Effect of 21 daily doses of 25 mg/kg OPLS on lymphocyte subsets in peripheral blood of rhesus macaques. Samples were drawn from the animals at baseline (day 0), at an intermediate time point during treatment (day 8) and 1 day after the termination of dosing (day 22) and immunophenotyped using flow cytometry. With the exception of a transient increase in the % of B-cells, no significant change was observed between baseline and post treatment levels. (* p value<0.05). The data indicate that OPLS does not alter the state of general immune-competence of the animals at the given dose.

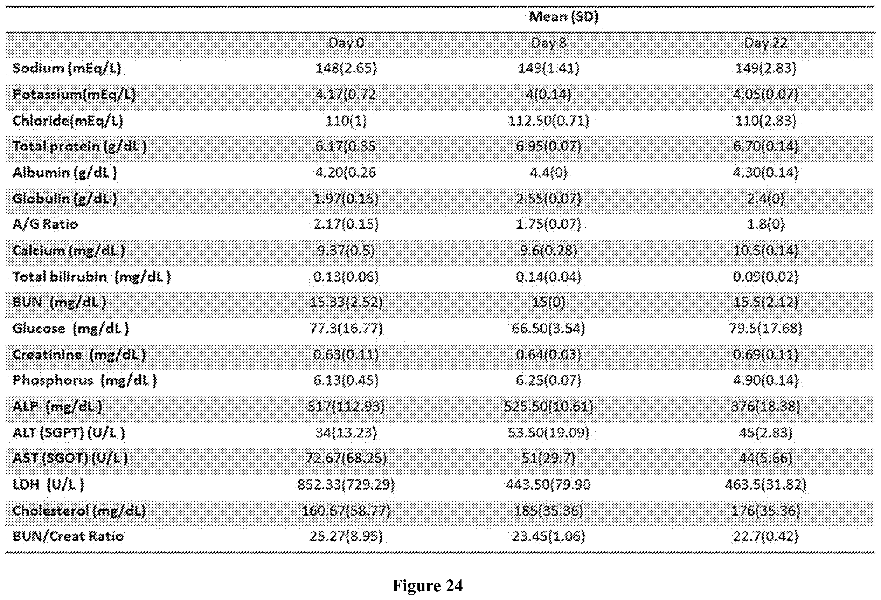

Figure 24. Effect of 21 daily doses of 25 mg/kg OPLS on plasma chemistry markers of rhesus macaques. Samples were drawn from the animals at baseline (day 0), at an intermediate time point during treatment (day 8) and 1 day after the termination of dosing (day 22). The data indicate that there is no impact of the given OPLS dose on renal and hepatic function.

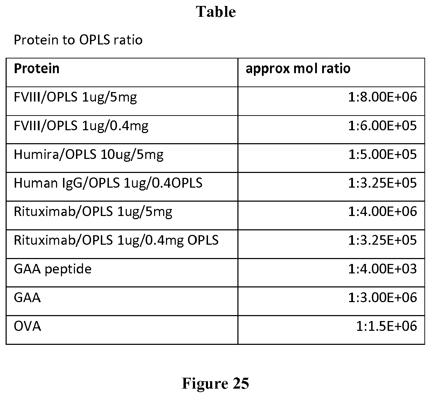

Figure 25. Table providing the mole ratio of protein to OPLS.
Figure 26. Week 6- Saphenous Sampling after administration of separate compositions of OPLS and GAA.
Figure 27. After Rechallenge- Terminal Sampling- Week 11.
Figure 28. Comparison from Week 6 to Week 11.
Figure 29: Plasma (open symbols) and lymph node (closed symbols) concentration-time profile of rituximab after iv (open circles) and sc (open diamonds) administration in SW mice. 1µg/g rituximab was injected sc or iv and the plasma concentrations were monitored for 120 hours. The sold line represents the fitting of the iv data to a 2 compartment model and the dashed line represent the fitting of the sc data to a model. The closed diamonds represents inguinal lymph node concentration of rituximab after sc administration. Values presented as mean ± SD.

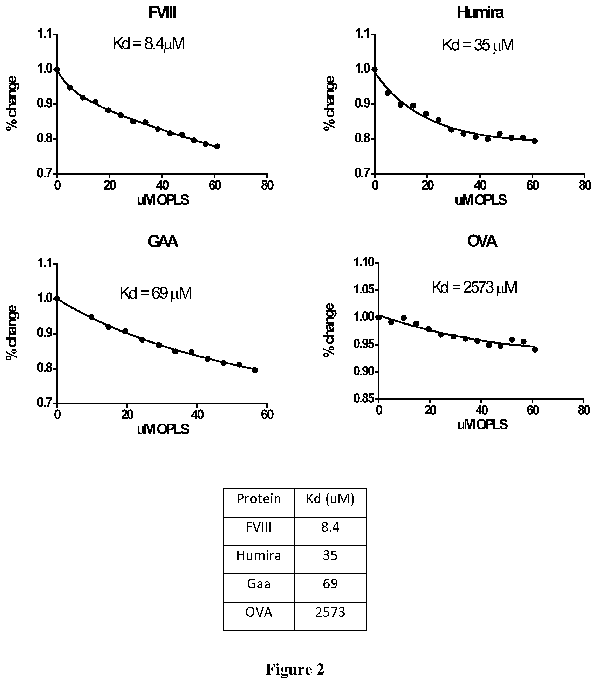

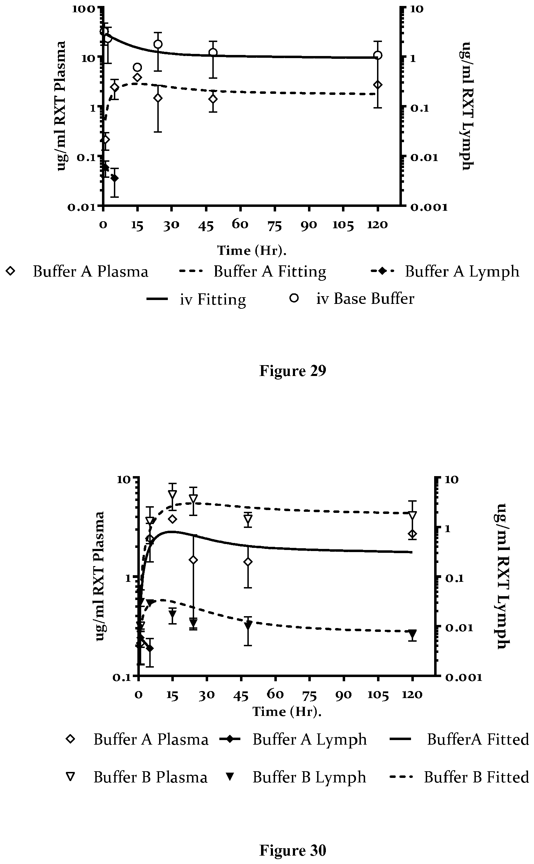

Figure 30. Plasma (open symbols) and lymph node (closed symbols) concentration-time profile of rituximab administered sc in isotonic buffer (buffer A open diamonds) and hypertonic buffer (buffer B open inverted triangle). Lymph node concentration of rituximab administered sc in isotonic buffer (buffer A closed diamonds) and hypertonic buffer (buffer B closed inverted triangles). Values presented as mean ± SD. The solid lines represent the fitting of the data to the model shown in figure 2. This data show increase plasma and lymph node exposure after sc administration of rituximab in hypertonic buffer as compared to isotonic buffer.

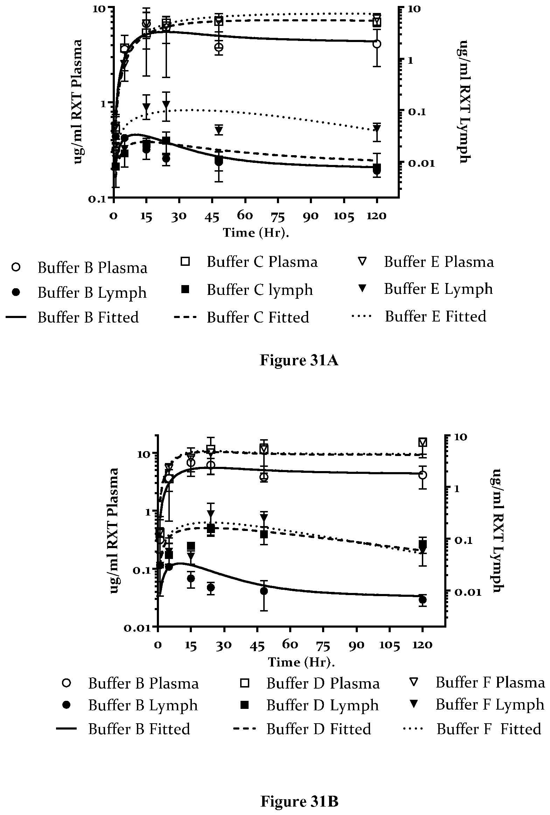

Figure 31. Plasma (open symbols) and lymph node (closed symbols) concentration-time profile of rituximab administered sc in different hypertonic buffers. 5.A Buffers C and E represent low and high dose of OPLS (20 mM open box) and 270 mM (open inverted triangle) as compared to hypertonic buffer containing NaCl only (buffer B open circles), with corresponding lymph node concentrations of rituximab for buffers C and E (closed box and closed inverted triangle respectively) as compared to hypertonic buffer containing NaCl only (buffer B closed circles). Values presented as mean ± SD. The solid lines represent the fitting of the data to a model. The data shows increase plasma concentration for rituximab administered with OPLS in a dose depended manner. This also corresponds to increase lymph node exposure especially for buffer E. Model predicts almost complete bioavailability for buffer E. 5.B Buffers D and F represent low and high dose of mannitol (20 mM open box) and 270 mM (open inverted triangle) as compared to hypertonic buffer containing NaCl only (buffer B open circles) with corresponding lymph node concentrations of rituximab for buffers D and F (closed box and closed inverted triangle respectively) as compared to hypertonic buffer containing NaCl only (buffer B closed circles). Values presented as mean ± SD. The solid lines represent the fitting of the data to a model. The data shows increase plasma concentration for rituximab administered with Mannitol. This also corresponds to increase lymph node exposure. Model predicts almost complete bioavailability for buffers D and F.
Figure 32. Anti-FVIII titers in HA mice re-challenged aggressively with 4 weekly sc injections of FVIII. Mice were pre-treated with FVIII alone, FVIII/OPLS or FVIII-Dexamethasone (Dex) sc for 4 weeks followed by a 2-week washout period, after which animals were re-challenged with FVIII alone. Animal in the FVIII and FVIII-Dex pre-treatment groups showed a robust anti-FVIII antibody response after re-challenge. The response was not as robust in the FVIII/OPLS pre-treatment group.

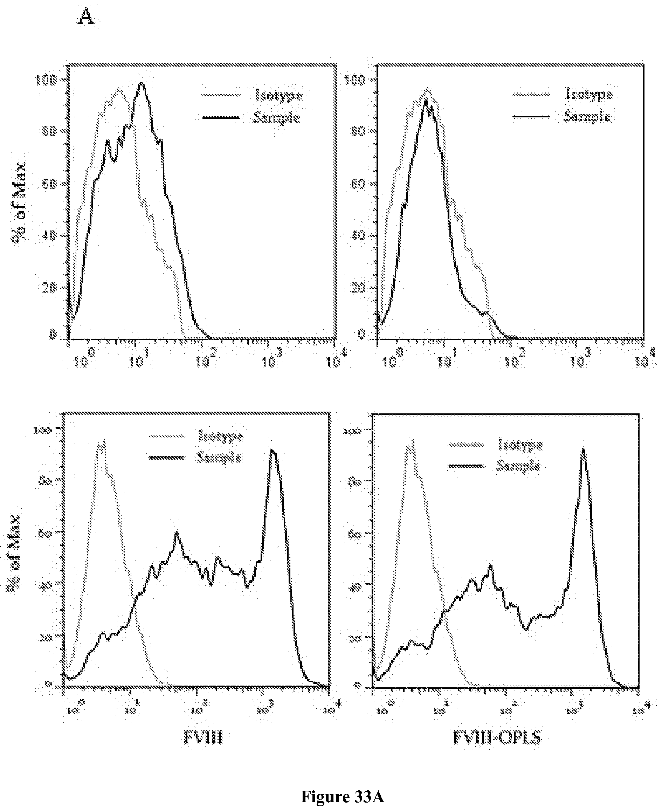

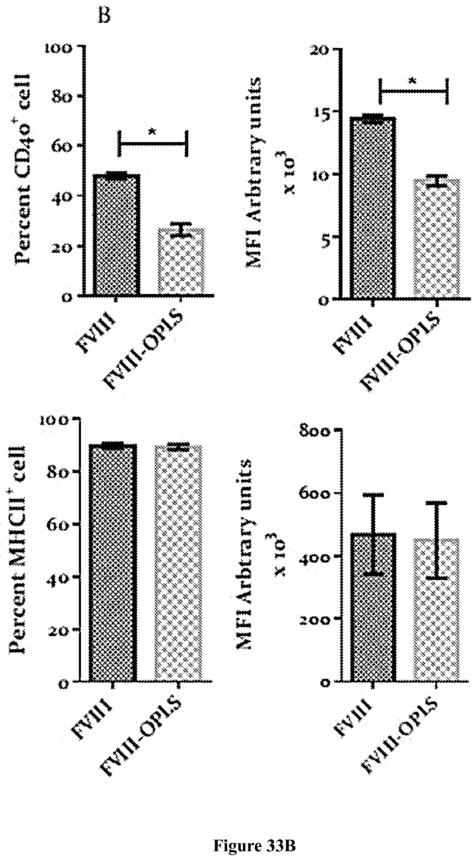

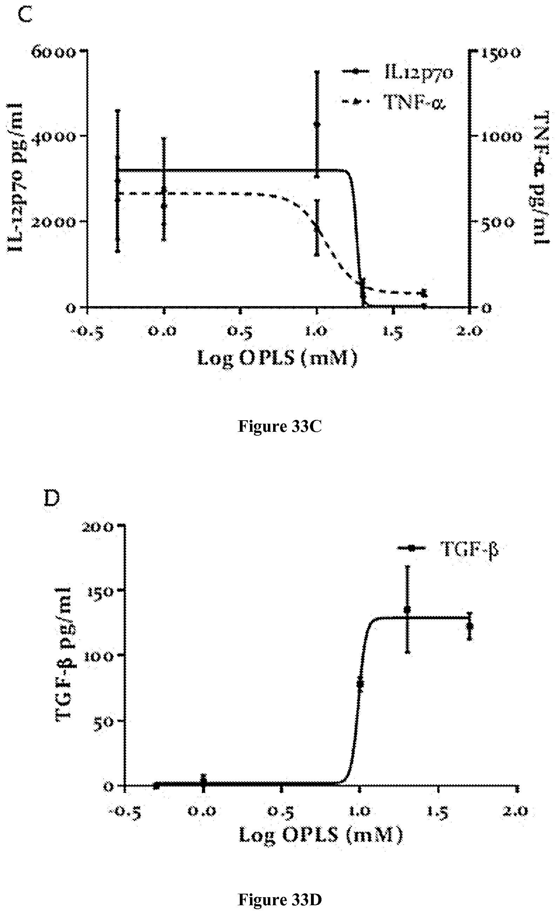

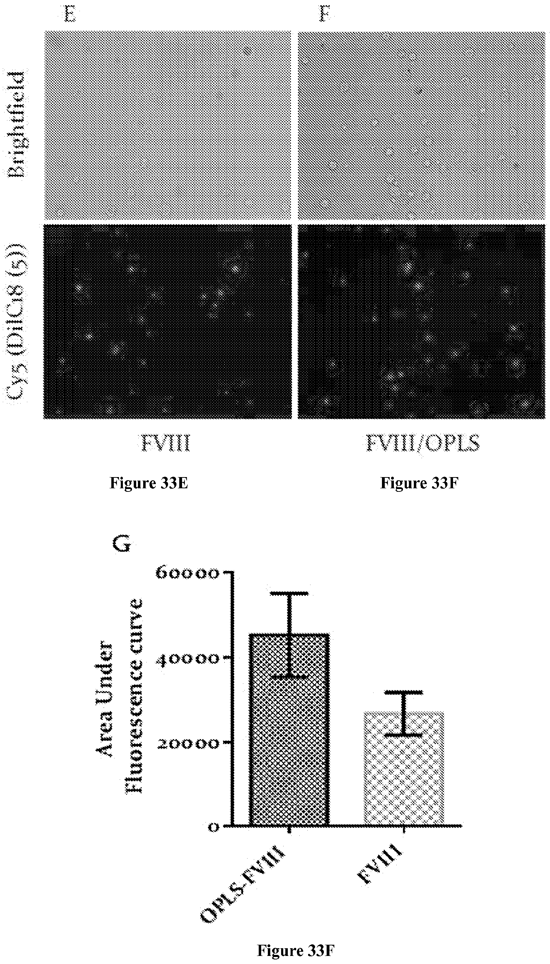

Figure 33. Characterization of BMDC exposed to FVIII in the presence and absence of OPLS. Panels A and B show the effect of 50 mM OPLS on phenotypic maturation of BMDC. Flow cytometry analysis of CD40 expression in BMDCs exposed to FVIII in the presence and absence of OPLS shows a significant reduction in CD40 expression in the OPLS treatment group. A representative data set is shown in upper panel (A), percent CD40+ cells and MFI presented in upper panel of 2B (n=3 *p<0.05 student t-test). MHCII expression in BMDC exposed to FVIII in the presence and absences of 50 mM OPLS is presented in the lower panels of figure 33A and 33B. The data shows no changes in MHCII expression levels in response to OPLS. Panels C and D show a dose dependent effect of OPLS on cytokine production by BMDC. BMDCs exposed to FVIII with increasing dose of OPLS showed a dose dependent decrease in pro-inflammatory cytokine TNF-α and IL-12p70 (panel C, n=4). This was accompanied by a dose dependent increase in the regulatory cytokine TGF-β (panel D, n=4). Panels E, F and G fluorescence labeling of BMDC and migratory potential of BMDCs treated ex vivo with FVIII alone or with 50 mM OPLS. Successful labeling of BMDC with DilC18(5) dye of FVIII (panel E) or FVIII/OPLS (panel F) groups. Area under the fluorescence curve for DilC18(5) labeled BMDC recovered in the inguinal lymph node of host HA mice (panel G).
Figure 34. OPLS curtail the immunological potential of BMDC exposed *ex vivo* to FVIII in host HA mice. Total anti-FVIII antibodies in host HA mice after 3 weekly sc administration of BMDCs exposed *ex vivo* to FVIII alone or FVIII/ OPLS (50mM). Data shows statistically significant reduction in anti-FVIII Titers in the OPLS treated group. Plasma from naive HA mice was used to calculate cutoff values for ELISA.
Figure 35. *In vivo* dose dependent response to OPLS/FVIII treatment. HA mice treated with high dose OPLS (5 mg) showed a statistically significant reduction in anti-FVIII titers as compared to FVIII alone and low dose OPLS (0.4 mg).

### DETAILED DESCRIPTION OF THE DISCLOSURE

The present disclosure provides compositions comprising OPLS or lipidic particles that, when administered in combination with or in a sequential manner with one or more antigens, provides for a reduction in immunogenicity of the antigen and an improvement in its pharmacokinetic properties and/or the induction of immune tolerance towards the antigen.

As used herein, the term biotherapeutics means biologically-based therapeutics, including recombinant proteins, peptides, therapeutic/prophylactic antibodies (e.g. monoclonal antibodies) and fragments thereof. Biotherapeutics, in this description, serve as and are sometimes referred to as an antigen.

The agent (such as a therapeutic agent) is delivered in conjunction with an immune tolerance inducing agent such as O-Phospho-L-Serine ("OPLS"); or lipidic particles which may be: (a) unilamellar or multilamellar liposomes comprised of phospatidylserine ("PS") and one or more phospholipids ("PS-liposomes"); or (b) non-liposomal structures comprised of serine including micelles, condensed structures, and cochleate. OPLS is available commercially. The phospholipids, including PS, can be obtained from various sources both natural and synthetic. For example, PS can be obtained from porcine brain PS or plant-based soy (soya bean) PS. Egg PC and PS and synthetic PC are also available commercially. The term lipidic compositions includes OPLS as well as lipidic particles.

The present disclosure provides compositions and methods for improving pharmacokinetic (PK) properties. In one embodiment, an "improvement" in pharmacokinetic properties means an improved half-life or bioavailability for the antigen. For example, when OPLS or lipidic compositions were subcutaneously administered with an antigen (such as Factor VIII), there was an improvement in the lymphatic uptake and the plasma PK profile relative to an administration of the free protein administered subcutaneously. Improvement in lymphatic uptake and plasma concentrations was also seen when using Rituximab as the antigen, providing further evidence that this is a broadly applicable approach.

In one embodiment, the immune tolerance inducing agent comprises, consists essentially of, or consists of OPLS. In one embodiment, the only immune tolerance inducing agent in the lipidic composition is OPLS. The OPLS concentration can be from 1 mM to 100 mM including all integers and ranges therebetween. In one embodiment, the OPLS amount is from 100 to 300 nM including all integers and ranges therebetween. In one embodiment, the mM concentration is 5, 10, 15, 20, 25, 30, 35, 40, 45, 50, 55, 60, 65, 70, 75, 80, 85, 90, 95, or 100. In another embodiment, the OPLS concentration is between 21 mM and 100 mM including all integers therebetween. In another embodiment, it is between 25 mM and 75 mM including all integers therebetween. In another embodiment, the OPLS concentration is between 35 mM and 65 mM including all integers therebetween. In yet another embodiment, the OPLS concentration is between 45 mM and 55 mM including all integers therebetween. In one example, the OPLS concentration is 50 mM. In one embodiment, the OPLS is 125, 150, 175, 200, 225, 250 or 275 mM. Alternatively, the amount of OPLS required to practice the methods disclosed herein can be described in terms of dose. The dose can be any amount above 1 mg. In one embodiment, the dose of OPLS can be from 1 mg to 1,000 mg including all integers therebetween. In a particular example, the dose is 5 mg.

In general, any suitable buffer may be used. For example, any phosphate buffer (including phosphate buffered saline) or TRIS buffer may be used. In one embodiment, the reconstitution medium described in U.S. patent no. 8,349,794 is used. The pH of the buffer may be from 5 to 8. In one embodiment, the pH of the buffer may be from 5.5 to 8 or 5.5 to 7.6, or 6.0 to 7.6. In another embodiment, the pH of the buffer may be from 6 to 8, or 7.2 to 7.6 or 7.4.

In one embodiment, the method for improving lymphatic uptake and/or plasma concentrations of a concomitantly or sequentially administered antigen is achieved through the use of a hypertonic solution comprising OPLS. In one embodiment, the compositions comprise, OPLS and NaCl. In one embodiment, the compositions comprise OPLS and mannitol, and optionally, NaCl. The increase in osmolality can be achieved by any combination of any amount of one of more of OPLS, mannitol and NaCl.

In another embodiment, the method for improving lymphatic uptake and/or plasma concentrations of a concomitantly or sequentially administered antigen is achieved through the use of a hypertonic solution (defined herein as having an osmolality of greater than 300 mmol/kg) comprising at least one of: OPLS and mannitol. In one embodiment, the osmolality of the solution ranges between 300 mmol/kg and 1,100 mmol/kg, including all integers and ranges therebetween. In one embodiment, the osmolality of the solution ranges between 300 mmol/kg and 800 mmol/kg, including all integers therebetween. In one embodiment, the osmolality is from 301 to 1,100 mmol/kg or from 500-600 mmol/kg. In one embodiment, the osmolality is from 290 to 300 mmol/kg (including all integers and ranges therebetween). In specific embodiments for hypertonic solutions comprising OPLS, wherein the osmolality of the solution ranges between 300 mmol/kg and 1,100 mmol/kg, higher concentrations (e.g. >100 mM) of OPLS result in greater lymphatic uptake and/or plasma concentrations that lower concentrations (e.g. <100 mM) of OPLS. Similarly, in specific embodiments for hypertonic solutions comprising mannitol, wherein the osmolality of the solution ranges between 300 mmol/kg and 1,100 mmol/kg, higher concentrations (e.g. >100 mM) of mannitol result in greater lymphatic uptake and/or plasma concentrations than lower concentrations (e.g. <100 mM) of mannitol.

Note that although lipidic compositions comprising, consisting essentially of or consisting of O-Phospho-D-Serine ("OPDS") were used in certain examples disclosed herein, effects similar to those provided using OPLS were observed. While not intending to be bound by any particular theory, it is considered that the observed effects of OPDS, at least in part, are due to the fact that OPDS is very unstable and that a portion of OPDS is getting converted to OPLS *in vivo.*

In another embodiment, the lipidic particles are liposomes comprised of phosphatidylserine ("PS") and phosphatidylcholine ("PC") and, optionally, cholesterol (sometimes referred to as "PS/PC"). In one embodiment, the phospholipids in the lipidic compositions comprise or consist essentially of, or consist of PS and PC present in the ratio of 10:90 to 30:70 and all ratios therebetween. In one embodiment, the only phospholipids in the lipidic particles are PS and PC as described above. In some embodiments cholesterol may also be present, but is not necessary to practice the methods disclosed herein. However, in cases where the antigen is complexed (vs. co-administered) with the lipidic particle and release of the antigen is desired, it is preferable to include 1-33% cholesterol (percent of PS and PC together).

In other embodiments, the phospholipids in the lipidic compositions comprise or consist essentially of, or consist of PS, phosphatidylinositol ("PI") and PC and, optionally, cholesterol (sometimes referred to as "PS/PI"). The combination of PS and PI is preferably not more than 60 mol % and PC is at least 40 mol %. The PI component of the PS/PI liposomes is from 1 mol % to 30 mol % and all integer mol % values there between. For PS liposomes (PS/PC and PS/PI liposomes), the PS should be at least 1 mol %. In one embodiment, PS is at least 10 mol %.

PS, PC and PI may have from 0-22 carbon atoms in the acyl chain. For example, when the carbon atom is 0 for PS, the molecule is O-Phospho-L-Serine (OPLS). In one embodiment, the acyl chains have 1 to 22, 2 to 22, or 6 to 22 carbons (and all integer number of carbons and ranges therebetwen). The acyl chains can be saturated or unsaturated and may be same or different lengths. Some examples of acyl chains are: Lauric acid, Myristic acid, Palmitic acid, Stearic acid, Arachidic acid, Behenic acid, Oleic acid, Palmitoleic acid, Linoleic acid, and Arachidonic acid. The PC may have one or two acyl chains. For example, the PC may include lysophosphatidylcholine (LPC). For example, in one embodiment, the composition of the PS liposomes was PS:LPC:PC as 30:10:70 mol%. The LPC can be 0 mol % to 20 mol %. In one embodiment, it is 1 mol % to 20 mol % and all integer mol % values and ranges therebetween.

The lipidic particles of the present disclosure can be prepared by thin lipid film hydration using the appropriate molar ratios of PS, PC and, optionally, cholesterol; or appropriate ratio of PS, PC and PI, and optionally cholesterol, in a suitable buffer. The lipids are dissolved in chloroform and the solvent is dried. The resulting multilamellar vesicles (MLVs) are extruded through the desired size filters (sizing device) under high pressure to obtain lipidic structures of the present disclosure. In one embodiment, the size of 50, 60, 70, 80, 90, 95 or 100% (including all percentages between 50 and 100) of the lipidic particles is from 40 nm to 4 micron including all sizes therebetween in the nanometer and micrometer range. In another embodiment, size of the particles is from 60 to 140 nm. In one another embodiment the particles are less than 140 nm (as calculated from micrographs and dynamic light scattering measurements) so that the particles are not filtered out in the Reticulo Endothelial System (RES) so as to become available for the immune system reaction. Thus in one embodiment, at least 50% of the particles are less than 140 nm. For example, the particles are less than 120 nm such as from 40 and 100 nm. In various embodiments, 50, 60, 70, 80, 90, 95 or 100% of the particles are less than 140 nm such as from 40 and 100 nm or 60 to 100 nm.

Preparation of lipidic compositions comprising, consisting essentially of, or consists of OPLS, can be as simple as a buffer preparation. The desired amount of OPLS can be weighed out and added to any buffer. The pH of such OPLS buffer solution can then be adjusted to a desired value (such as 7.4) using methods known in the art.

The antigen may be a peptide (generally 50 amino acids or less) a polypeptide (generally 100 amino acids or less) or proteins (larger than 100 amino acids). The agent may be a therapeutic antigen or may be an antigen against which an individual is already primed, but against which immune tolerance is desired (such as in allergic reactions or transplant situations). The proteins or peptides may be neutral or charged (negatively or positively). Such proteins include proteins involved in the blood coagulation cascade including Factor VIII (FVIII), Factor VII (FVII), Factor IX (FIX), Factor V (FV), and von Willebrand Factor (vWF), von Heldebrant Factor, tissue plasminogen activator, insulin, growth hormone, erythropoietin alpha, VEG-F, Thrombopoietin, lysozyme, acid alpha glucosidase (GAA), therapeutic antibodies, including monoclonal antibodies such as adalimumab, myelin sheath proteins, myelin oligodendrocyte glycoprotein (MOG), glutamic acid decarboxylase 65 (GAD), insulin receptor, and the like. In one embodiment, the antigen is a monoclonal antibody or fragment thereof (such as Fc portion)." In one embodiment, the antigen can be one that ordinarily provokes a relatively mild allergic reaction, such as would typically be caused by pollen, animal dander, house dust and the like, or an antigen that ordinarily would provoke in the individual a severe allergic reaction, such as components in venom from insect stings, nut allergens, certain antibiotics, and other compositions that can cause severe allergic responses in the particular individual in question or may be a transplant relevant antigen.

In some embodiments it is desirable to associate (i.e. complex) the antigen and the lipidic composition. To effect association (i.e. form a complex) of the antigen with the lipidic structures, the antigen in a suitable buffer is added to the lipidic structures. The free antigen is then separated from the lipidic structures by centrifugation methods such as density gradient centrifugations. In various embodiments, the association efficiency of the antigen with the lipidic particles is at least 30, 40, 50, 55, 60, 65, 70, 75, 80, 85, 90 and 95%. If desired, the lipidic particles with the associated antigen can be lyophilized for future use. In one embodiment, the lipidic structures of the present disclosure prior to association with the antigen can be lyophilized and stored. When needed, the lipidic structures can be reconstituted and then used for combination with antigen to effect association of the antigen with the lipidic structures prior to use.

In embodiments where the antigen is associated with the PS-liposomes, the association can be such that the molar ratio between the antigen to lipid is between 1:10 (antigen:lipid) to 1:30,000 (antigen:lipid) and all ratios therebetween. In one embodiment it is about 1:10,000 (antigen:lipid). As the therapeutic dose of an antigen increases, the ratio decreases. In other embodiments, the ratio is about 1:2,000 or 1:4,000 (antigen:lipid). The maximum antigen:lipid ratio of the present disclosure is 1:30,000. Since dosing for different proteins varies, the amount of dosing required will alter the ratio.

In some embodiments, the lipidic composition is not complexed with the antigen. In one embodiment, the lipidic particles of the present disclosure prior to administration can be lyophilized and stored. When needed, the lipidic particles can be reconstituted and then administered to an individual.

In embodiments where OPLS is used, the molar ratio between the antigen to OPLS can be from 1:1 to 1: 1X10⁷ and all ratios therebetween and all ranges therebetween. In one embodiment, it is from 1:1 to 1:1X10⁶ all ratios therebetween. In one embodiment, the ratio of antigen to OPLS is between 1:1 to 1:1,000 and all ratios therebetween.

Where we describe a complex or that an antigen and a lipidic composition are associated (i.e. complexed), it is meant that the K_{d} for the antigen and the lipidic composition is less than 10 µM. In one embodiment, the K_{d} is 8.4µM. Where we describe that an antigen and a lipidic composition are not complexed, we mean that the K_{d} for the antigen and the lipidic composition is greater than or equal to 10 µM. The range of bound to free is as follows: where the dissociation constant (Kd) is less than 10 µM the protein is considered bound or complexed; where the Kd is 10 uM to 500 uM, the protein is considered loosely bound with non-specific interactions; where the Kd is greater than or equal to 500uM the protein is unbound ('free"). In one embodiment, the Kd is greater than 10 uM. In one embodiment, the K_{d} can be from 10 µM to 5,000 µM including all integers therebetween. In various embodiments, the K_{d} is at least 10, 20, 30, 40, 50, 60, 70, 80, 90 and 100 µM. In other embodiments, the K_{d} is10, 15, 20, 30, 40, 50, 60, 70, 80, 90, 100, 150, 200, 300, 400, 500, 1000, 1500, 2000, 2500, or 3000 µM.

In some embodiments, at least 55%, 60%, 65%, 70%, 75%, 80%, 85%, 90%, 95% or 99% of the antigen is present as unbound or loosely bound to the lipidic composition (such as OPLS). For example, in embodiments, a composition may be administered via a cutaneous route, wherein the composition comprises a therapeutic agent that is loosely bound or unbound to OPLS.

In one embodiment, the OPLS or other lipidic composition may be combined with the antigen just prior to administration (such as less than 1 minute or within 1 to 5 minutes of the time of administration). In one embodiment, the combining is done at room temperature (such as from 20 to 26 °C and all integer temperatures therebetween, or less than 20 C), just prior to administration so that there is no, or, insignificant complexation of the OPLS and the antigen. In specific embodiments, the combined OPLS and antigen are not exposed to a temperature above 22, 23, 24, 25 or 26 °C so that there is no opportunity for complex formation.

The compositions described in this disclosure can be used for any individual (e.g., a human or a non-human mammal). For example, they can be administered to an individual who has previously been administered and/or exposed to the antigen but has not previously developed immune intolerance to said antigen. In yet another example, they can be administered to an individual who has previously been administered and/or exposed to the antigen and has developed immune intolerance to said antigen. The individual may or may not be showing indications of a recent immune intolerance. They are also useful for administration to naive individuals (i.e., those individuals who have not been administered and/or exposed to the antigen previously). Immune intolerance as used herein means the individual should have measurable (by standard methods such as ELISA or activity assays) antibody production. Conversely, a lack of immune intolerance means the individual has no measureable antibodies. By "administered" or "administration" is meant that the antigen and the lipidic particles are delivered to the individual or introduced into the individual's body by any means or route of delivery such as intravenous, intramuscular, intraperitonial, mucosal, subcutaneous, transdermal, intradermal, oral or the like. In one embodiment, the antigen and the lipidic particles are administered via a cutaneous route such as subcutaneously.

In one aspect, the disclosure provides a method for reducing the immunogenicity and/or improving the pharmacokinetic properties of an antigen comprising the steps of: a) administering to an individual, a composition comprising the antigen; and b) sequentially or concomitantly with the administration of the composition in a), administering to the individual a composition comprising one or more lipidic compositions disclosed herein. In one embodiment, the one or more lipidic compositions are selected from the group consisting of OPLS and PS liposomes, as defined herein. In one embodiment the PS liposomes are selected from the group consisting of PS/PC and PS/PI, as described herein. In one embodiment, the K_{d} for the antigen and the lipidic composition is greater than or equal to 10 µM. In one embodiment, one or both of lipidic composition and the antigen are delivered via a cutaneous route.

In other embodiments the method for reducing the immunogenicity and/or improving the pharmacokinetic properties of an antigen may be further comprise of one or both of the following: (i) obtaining a biological sample from the individual and measuring one or more of the following: T regulatory cells, TGF-β, IL-10, antigen-specific antibody titers, B cells, including antigen-specific memory B cells, IL-6, IL-17, CD40, CD80, and/or CD86, and/or (ii) following the administration of the antigen and OPLS or another lipidic composition, obtaining a biological sample from the individual and measuring one or more of the following: T regulatory cells, TGF-β, IL-10, antigen-specific antibody titers, B cells, including antigen-specific memory B cells, IL-6, IL-17, CD40, CD80, and/or CD86. In embodiments where both (i) and (ii) are practiced, levels of the cells and/or biomarkers described above may be compared to measure the effectiveness of the method.

In still other embodiments, the method for reducing the immunogenicity and/or improving the pharmacokinetic properties of an antigen (such as FVIII, GAA and adalimumab) described above may be repeated 1 time per week to three or more times per week either indefinitely or for a period of 1, 2, 3, 4, 5, 6, 7, 8, 9, 10, 11, 12 weeks or more weeks in an effort to achieve a greater degree of immune tolerance to develop. After a suitable period of time (such as at least 4 days after the last primer), the individual can be administered a second composition (one or more administrations) comprising the antigen in the free form, complexed to or in combination with lipidic particles having a different composition than the composition previously administered (the "priming composition"), or complexed (covalently or noncovalently) to PEG, or surfactants or microspheres and the like. The lipidic structures may include micelles, cochleates, lipidic molecular assemblies and the like. In one embodiment, the individual is administered the second composition (which may be a therapeutic composition) after 4 to 30 days, including all integer number of days and ranges therebetween, of the last priming composition administration. In one embodiment, the individual is administered the second composition after 5, 6, 7, 8, 9, 10, 11, 12, 13, 14 and 15 days of the last of the last priming composition administration. Less immunogenic formulations are disclosed in U.S. patent and patent application nos. 7,351,688; 7,875,288; 7,875,289; 7,625,584; 20090053297; PCT/US2010/041196.

The phospholipid composition of the lipidic particles in the second composition (which may be a therapeutic composition) may be the same or different from the phospholipid composition of the lipidic particles of the first composition (which is a priming composition). The phospholipid composition change may include the type of phospholipid or the ratio of different phospholipids. In one embodiment, the therapeutic composition includes LPC and sphingosine.

In one embodiment, the frequency of administration of the priming composition is once-a-week for four weeks via the subcutaneous route. In one embodiment, at least four weekly administrations are used to induce tolerance. Administrations of the antigen and lipidic compositions may take place either concomitantly or sequentially, as described herein. More doses of antigen in combination with or complexed to either PS-liposomes or OPLS can be used if necessary.

Similar to the current practice of administering "booster" vaccines, this method can be repeated in the event said individual becomes immunologically responsive over time following future exposures to said antigen.

In another aspect, the present disclosure provides a method for administering an antigen (a) without inducing an increase in antigen-specific B memory cells or (b) which reduces the number of antigen-specific B memory cells in an individual comprising the step of administering to the individual an antigen and one or more lipidic compositions. In one embodiment, the antigen and/or lipidic composition are administered via cutaneous route. For example, one or both of OPLS and the antigen compositions may be administered subcutaneously, intra- or trans-dermally, and/or via topical route. In one embodiment the one or more lipidic compositions are selected from the group consisting of OPLS and PS liposomes. In one embodiment the PS liposomes are selected from the group consisting of PS/PC and PS/PI, as described herein. In some embodiments, the antigen and the one or more lipidic compositions are present in the same formulation. In other embodiments, the antigen and the one or more lipidic compositions are present in different formulation. In embodiments where the antigen and the one or more lipidic compositions are present in the same formulation, the K_{d} for the antigen and the one or more lipidic compositions is greater than or equal to 10 µM. Administration of the lipidic composition and the antigen may take place either concomitantly or separately.

In embodiments where the antigen and lipidic compositions are administered concomitantly, the lipidic composition and the antigen may be present within the same formulation or within different formulations.

In embodiments where the antigen and lipidic composition are present within the same formulation, they may be either complexed or not complexed. In one embodiment, the antigen and the lipidic component (such as OPLS) are loosely bound or are unbound).

In embodiments where the antigen and lipidic compositions are administered sequentially, either the antigen or the lipidic composition may be administered first provided that whichever component is administered second is administered within close proximity to the first, both in terms of space and time, so as to allow them to be presented together. The sequence of administration is irrelevant. In one embodiment, the antigen and the lipidic composition are administered at anatomical sites that are less than 2 inches apart from one another. Administration of the second component can be carried out within 0.5, 1, 1.5, or 2 inches from the site of administration of the first component. In one embodiment, the antigen and the lipidic composition are administered within 24 hours from one another. Administration of the second component can be carried out within 0.5, 1, 5, 10, 15, 20, 25, 30, 35, 40, 45, 50, 55, 60, 120, 240, 300, 480, 600, 720, 1080 or 1440 minutes from the time the first component is administered. In a preferred embodiment, they are administered via the same or similar cutaneous routes. For example, both administered subcutaneously or the antigen administered subcutaneously and the lipidic composition administered transdermally.

In one embodiment, the antigen (e.g. protein, peptide or polypeptide) is administered subcutaneously (such as by subcutaneous injection) and OPLS is delivered to the same or nearby site by topical route. For topical or transdermal delivery, OPLS may be mixed under sterile conditions with a pharmaceutically-acceptable carrier, and with suitable preservatives, buffers, or propellants. The topical formulations may also contain additional excipients, such as animal and vegetable fats, oils, waxes, paraffins, starch, tragacanth, cellulose derivatives, polyethylene glycols, silicones, bentonites, silicic acid, talc and zinc oxide, or mixtures thereof. Topical formulations may be liquids, emulsions, sprays, or foams. The formulations may also contain additional excipients such as lactose, talc, silicic acid, aluminum hydroxide, calcium silicates and polyamide powder, or mixtures of these substances. Sprays or foams can additionally contain customary propellants, such as chlorofluorohydrocarbons and volatile unsubstituted hydrocarbons, such as butane and propane. In one embodiment, dermal patches may be used. These have the added advantage of providing controlled delivery to the site. Such dosage forms can be made by dissolving or dispersing OPLS in a suitable medium and applying the formulation to a portion of the patch. Absorption enhancers can be used to increase the flux of OPLS into and across the skin. The rate of such flux can be controlled by either providing a rate controlling membrane or dispersing the active ingredient in a polymer matrix or gel. In one embodiment, the OPLS compositions are applied to dermal patches, bandages, adhesive bandages, gauges or other similar materials that can be directly applied to the desired area. A portion of the dermal patch (such as an adhesive bandage) on which OPLS composition is present may be protected by a sealed, removable covering until use. The adhesive surfaces may also be protected by the same or different removable covering. At the time of use, the covering or coverings can be removed and the strip or patch applied to the area where delivery of the OPLS to or through the skin in desired.

In one aspect of the disclosure are provided kits useful for reducing immunogenicity of an antigen, or improving the pharmacokinetic properties of an antigen, or reducing the number of antigen-specific B memory cells. In one embodiment, the kit comprises OPLS and instructions for administration of OPLS in conjunction with an antigen, and optionally, administration devices for administration of the OPLS and/or the antigen. The kit may also optionally comprise one or more antigens. The instructions for administration of OPLS and the antigen may indicate that these should be administered via a cutaneous route. The instructions may also indicate the site of administration and temporal or spatial separation of the administrations. In one embodiment, the instructions may also indicate that the OPLS and the antigen may be combined just prior to administration (such as within 1 minute or within 1 to 5 minutes of the administration), and the temperature range at which the OPLS and the antigen may be combined (such as from 20 to 26 °C).

In one embodiment, a kit comprises the antigen (e.g. protein, peptide or polypeptide) component and the lipidic composition component. The kit may also comprise a set of instructions for preparation and use of the components. In one embodiment, the lipidic composition is selected from the group consisting of OPLS and PS liposomes. In one embodiment, the PS liposomes are selected from the group consisting of PS/PC (with optionally PE, where the PC concentration is 1-10 mol% and PE mole% displaces PC amount) and PS/PI, as described herein. In one embodiment, the kit comprises an antigen (such as Factor VIII) in a lyophilized form, a vial of reconstitution medium for preparing the antigen and/or the lipidic composition (such as OPLS) for administration (such as subcutaneous administration), a lipidic composition delivery article, and a set of instructions for storage, preparation and/or use of the various components. In some embodiments, the reconstitution medium is comprised of lipids selected from the group consisting of phosphatidylserine, phosphatidylcholine, and combinations thereof. In other embodiments, the reconstitution medium is an OPLS solution. The reconstitution medium is a buffer solution, however the reconstitution medium as defined in this disclosure may or may not contain lipids. If the reconstitution medium does not contain lipids, then the other vial in the kit will provide the requisite lipid. If this is the case, it can be any phosphatidylserine-containing solution, including OPLS. The lipidic composition delivery article may be a container with a liquid or lyophilized formulation comprised of the lipidic composition (such as OPLS and PS liposomes), a syringe prepackaged with the lipidic composition, a spray or foaming can containing the formulation comprised of the lipidic composition for topical application, or a topical patch comprising the formulation comprised of the lipidic formulation. In embodiments where topical application is to be practiced, OPLS is the preferred lipidic composition due to its ability to penetrate the skin. The set of instructions may be printed materials or electronic information storage medium such as thumb drives, electronic cards, and the like. The set of instructions may provide instructions regarding site, sequence and frequency of administration of the components and any other information relevant to the intended use. The various components of the kit may be provided in separate vials or containers within the kit, or as one unit (such as in separate compartments in a one blister packaging).

In one embodiment, this disclosure provides a method for inducing immune tolerance toward an antigen comprising: a) providing a first composition comprising OPLS, which is substantially free of the antigen, and a second composition comprising the antigen, which is substantially free of OPLS, and b) administering the first and the second compositions separately through a cutaneous route to an individual; wherein the two compositions are administered at sites that are within 2 inches of each other. The antigen and OPLS may be administered in isotonic solution (270 to 300 mmole/kg) or in hypertonic solutions (greater than 300 mmol/kg). In various embodiments, the agent may be a therapeutic, which may be present at therapeutic or sub-therapeutic dose. One or both compositions may be administered once or more weekly for 1 to 12 weeks or more. The term "substantially free" as used in this disclosure means that the amount of the particular materials present in the composition is not sufficient to have any detectable clinically relevant biological effect. For example, a composition of OPLS is substantially free of an antigen if less than 0.5 mole% of the antigen is present. Similarly, a composition of the antigen is substantially free of OPLS (or other lipidic components) if less than 0.5 mole% of OPLS (or a lipidic component) is present. In some embodiments, less than 0.4, 0.3, 0.2, 0.1, 0.05 mole% of OPLS or antigen are present in the compositions of the antigen or OPLS respectively.

In one embodiment, this disclosure provides a method for inducing immune tolerance toward an antigen comprising administering to an individual a composition comprising OPLS and a sub-therapeutic amount of antigen. The OPLS and the sub-therapeutic dose of antigen may be in separate compositions or the same composition. When present in the same composition, the OPLS and the antigen are such that they are loosely bound or unbound. The compositions may be delivered via the same or different cutaneous routes. A sub-therapeutic dose of a therapeutic antigen is defined as a dose that is at least 50% less than (i.e., it may be 1/2 of) the lowest acceptable therapeutic dose. The therapeutic doses of various therapeutics are well known to clinicians and those skilled in the art. In various embodiments, the sub-therapeutic dose may be any fraction from 1/2 to 1/100^{th} of the therapeutic dose. In one embodiment, it may be from 1/100^{th} to 1/500^{th} of a therapeutic dose. As an example, a therapeutic dose for FVIII is considered to be 40 IU/kg to 400 IU/kg, and a sub-therapeutic dose is 20 IU/kg and lower. In one embodiment, a sub-therapeutic dose is about 10 IU/kg. As another example, the clinical or therapeutic dose of GAA is 20 mg/kg, while a sub-therapeutic dose is 10 mg/kg. In one embodiment, the sub-therapeutic dose is 1 µg to 1 mg/kg.

In one embodiment, this disclosure provides a method for inducing immune tolerance toward an antigen comprising administering to an individual a composition comprising OPLS and a therapeutic amount of antigen. The OPLS and the therapeutic dose of antigen may be in separate compositions or the same composition. When present in the same composition, the OPLS and the antigen are such that they are loosely bound (K_{d} of 10 µM to 500 µM or unbound (K_{d} of greater than 500 µM). The compositions may be delivered via the same or different cutaneous routes. In one embodiment, the agent is an antibody, which may be a polyclonal antibody, a monoclonal antibody, a chimeric antibody, a fragment of an antibody, or combinations thereof.

In one embodiment, the present disclosure provides a method for inducing immune tolerance toward an antigen in an individual comprising a) obtaining dendritic cells from the individual; b) contacting the dendritic cells with OPLS and the antigen in vitro; and c) returning the dendritic cells to the individual. The dendritic cells may be exposed to OPLS and the antigen at the same time or sequentially. The antigen may be used at a therapeutic dose or a sub-therapeutic dose. In one embodiment, the dendritic cells may be exposed to more than one antigens.

In one aspect, the present disclosure provides kits for inducing immune tolerance. The kits may comprise one or more of the following: a composition comprising OPLS (which is substantially free or completely free of the antigen); a composition comprising one or more antigens (which is substantially free or completely free of OPLS or other lipid components); instructions for administration via a cutaneous route, or instructions for exposing cells in vitro; optionally, one or more administration devices for administration of the compositions. The administration devices may be syringes, injection pens, dermal patches and the like.

The following examples are presented to illustrate the present invention. They are not intended to be limiting in any manner. Example 14 is in accordance with the present invention. Examples 1 to 13, 15 and 16 are reference examples.

### EXAMPLE 1

### This example provides characterization of administered OPLS antigen compositions.

Materials: Full length recombinant human FVIII (Baxter Biosciences, Carlsbad, CA) was obtained as a gift from the Western New York Hemophilia Foundation. Ova and human IgG were from Sigam (St. Louis, MO) Humira and Rituximab are commercially available. For the present disclosure, Humira was obtained commercially, from a pharmacy. O-Phosphso-L-Serine (OPLS) was purchased from Sigma (Sr. Louis, MO). All solvents and buffer salts were obtained from Fisher Scientific (Fairlawn, NJ) or from Sigma (St. Louis, MO). Anti-FVIII monoclonal antibodies ESH8 were obtained from American Diagnostica Inc. (Greenwich, CT). Goat anti-mouse IgG was purchased from Southern Biotechnology Associates, Inc. (Birmingham, AL). Bovine serum albumin (BSA) was purchased from Sigma (St. Louis, MO) while the phosphatase substrate system (diethanolamine buffer and *p*-nitrophenyl phosphate) was purchased from KPL Inc. (Gaithersburg, MD). Chromogenix Coamatic FVIII assay kit was purchased from DiaPharma Group (West Chester, OH) for the detection of FVIII in plasma. anti-Ova IgG titier analysis kit was from alphadiagnostic international.

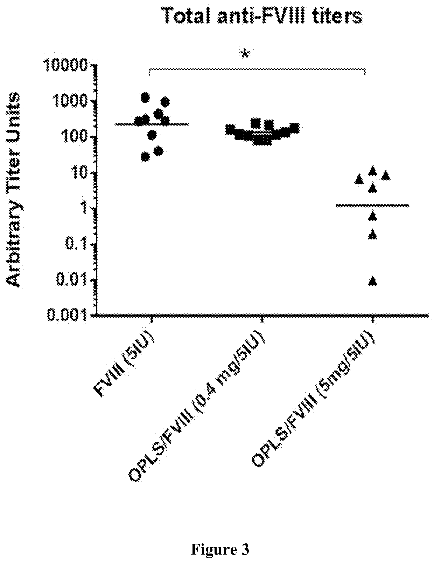

Effect of OPLS on the immunogenicity of FVIII after SC administration *in-vivo:* 21 HA mice between 6 and 8 weeks of age were divided into 3 groups: group one received 1 ug FVIII SC in the abdominal region. Group two received 1ug FVIII plus 0.4 mg OPLS SC in the abdominal region and group 3 received 1ug FVIII plus 5mg OPLS sc in the abdominal region. Incubation of FVIII with OPLS at 37C for 30 minutes ensures complexation, but for this example, OPLS and FVIII were combined at the desired concentrations at room temperature (22 °C) and immediately administered (within 1 minute). This treatment was given one day 1, 8, 10 and 12. Three days after the last injection the animals were sacrificed. Blood was centrifuged at 7500 rpm for 5 min at 4 degrees Celsius. Plasma was then removed and stored at -80 degrees Celsius until analysis. The results are shown in Figure 3.

Total titers were determined by classical standard ELISA. Anti-FVIII antibody (ESH8) was used as a control. Statistics was done on the arithmetic mean of the log transformed data, graphs are reported as geometric means. Non-responders were given the value of the plate specific factor for uniformity.

PK of FVIII in HA mice in the presence and absence of O-Phospho-L-Serine: 60 HA mice between 6 and 8 weeks of age where divided into 2 groups of 30 animals each. First group received 5IU/g FVIII in the abdominal region with massaging for 60 second after each injection. The second group received FVIII plus OPLS (5IU/gFVIII plus 0.4mgOPLS) in the abdominal region followed by massaging as above. 3 animals from each group were sacrificed at the following pre-set time point 4,6,9,12,15,18,24,33 and 44 Hours. Total blood as well as the inguinal lymph nodes were collected. Blood was centrifuged at 7500 rpm for 5 min at 4 degrees Celsius. All samples were stored at -80 degrees Celsius until analysis. Samples were analyzed using Coamatic Factor VIII kit (DiaPharma West Chester, OH, USA) assay according to manufacturer's instruction.

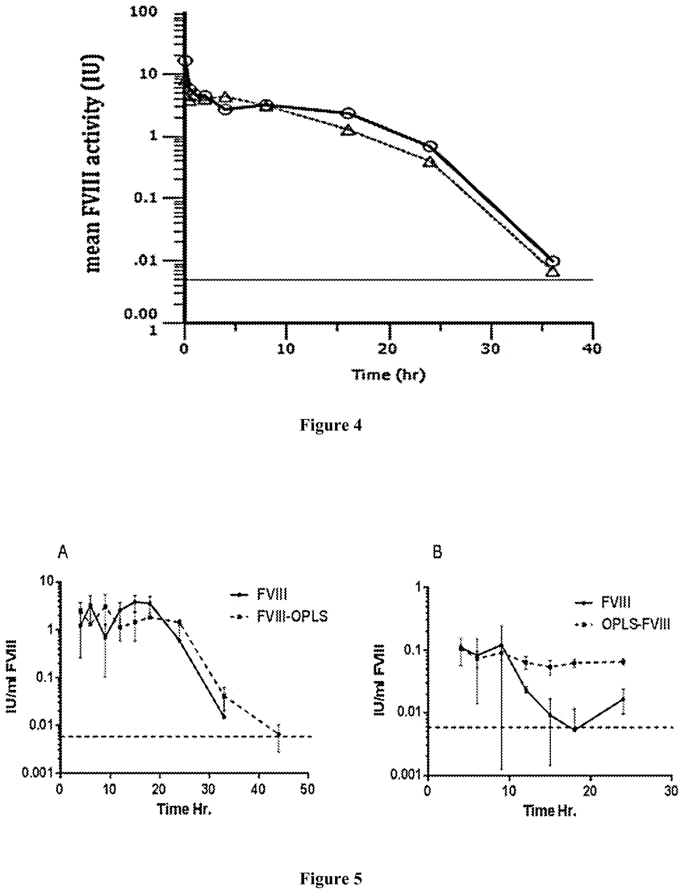

Intravenous co-administration of OPLS and biotherapeutic (e.g. FVIII) has little effect on the pharmacokinetics: only a slight increase in plasma exposure was seen (Figure 4). However, subcutaneous co-administration of biotherapeutics with OPLS or PS-liposomes improves lymphatic uptake from the subcutaneous space, which is a key uptake pathway for large proteins, and improves their bioavailability compared to subcutaneous administration of the biotherapeutic alone. When administered s.c., OPLS is able to effectively stop biotherapeutics from being rapidly cleared from the body. Improved lymphatic uptake and prolonged residence time in the lymphatics translates to prolonged plasma exposure (Figure 5).

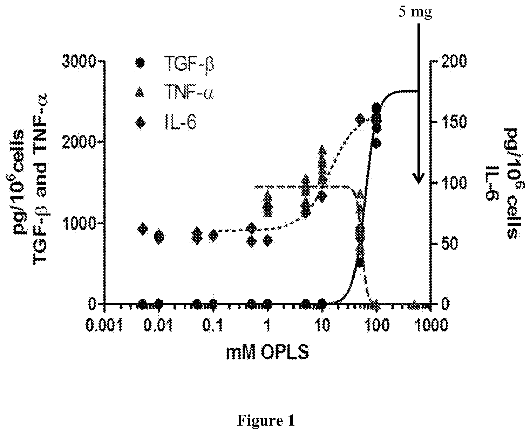

Effect of OPLS on the cytokine profile of Bone Marrow Derived Dendritic Cells (BMDC) in presence and absence of protein antigen (FVIII): Bone marrow was isolated from 2 HA mice between 6 and 8 weeks of age. Briefly, bone marrow was cultured in RPMI media supplemented with Fetal Bovine Albumin, 2-Macraptoethanol, Pen/Strip and GMCSF. Media was changed on days 3,6 and 8 of culture and DC's were used on day 9 or 10. This prolonged culturing method results in 70-80% immature DC's on day 9 or 10. Once the DC are harvested they are exposed to one of the following treatment groups: Naive (negative control), FVIII alone (positive control), FVIIII with increasing dose of OPLS 10 mM, 50 mM and 100 mM OPLS, OPLS alone (100nM), and Lipopolysacarides (positive control for inflammation response). Cells were platted in triplicate and 2 sets were made. One was used for cytokine analysis and was allowed to remain in culture for 72 hours before media was collected for cytokine analysis. TGF-β, TNF-α and IL-6 concentrations were determined using standard ELISA (DuoSet ELISA kit, R&D System Inc. Minneapolis, MN, USA). The other set was allowed to stay in culture for 24 house before media was removed and the DC's were co-cultured with CD4⁺ T-cells isolated from spleens of HA mice exposed to an aggressive immunization schedule with FVIII as follow: week 1: Monday 1 ug of FVIII given SC. Week 2: Monday, Wednesday and Friday 1 ug of FVIII given SC on each of those days. 72 hours after the last immunization the animals are sacrificed and spleens are collected, crushed and CD4⁺ T-cell were isolated using a negative isolation kit (Miltenyi Biotec, Auburn, CA, USA) as per the manufacturer's protocol. After 72 hours of co-culturing with DC, the supernatant is removed and cytokine analysis was done using standard ELISA to determine TFG-β and IL-17 production by T-cells. (DuoSet ELISA kit, R&D System Inc. Minneapolis, MN, USA). The results are shown in Figure 1.

The binding of OPLS to several therapeutic proteins was evaluated using fluorescence spectroscopy (Figure 2). The fluorescence emission reduced as the concentration of OPLS was increased. The fluorescence intensity changes were highest for FVIII compared to GAA and OVA. The binding constant estimated based on the spectroscopic titration showed that FVIII binds to OPLS with highest affinity whereas GAA and OVA with lowest affinity. The high affinity binding for FVIII is consistent with the existence of a putative lipid binding domain.

### EXAMPLE 2

### This example describes the effect of OPLS on the immunogenicity of ovalbumin (Ova) after SC administration in-vivo:

Methods: 14 SW mice between 6 and 8 weeks of age were divided into 2 groups: group one received 1 ug OVA SC in the abdominal region. Group two received 1ug OVA/5 mg OPLS SC in the abdominal region. This treatment was given one day 1, 8, 10 and 12. Three days after the last injection the animals were sacrificed. Blood was centrifuged at 7500 rpm for 5 min at 4 degrees Celsius. Plasma was then removed and stored at -80 degrees Celsius until analysis Titers values were obtained using an anti-Ova IgG titier analysis kit as per the manufacturers instruction (alphadiagnostic international). Statistics was done on the arithmetic mean of the log transformed data, graphs are reported as geometric means. Non-responders were given the value of the plate specific factor for uniformity.

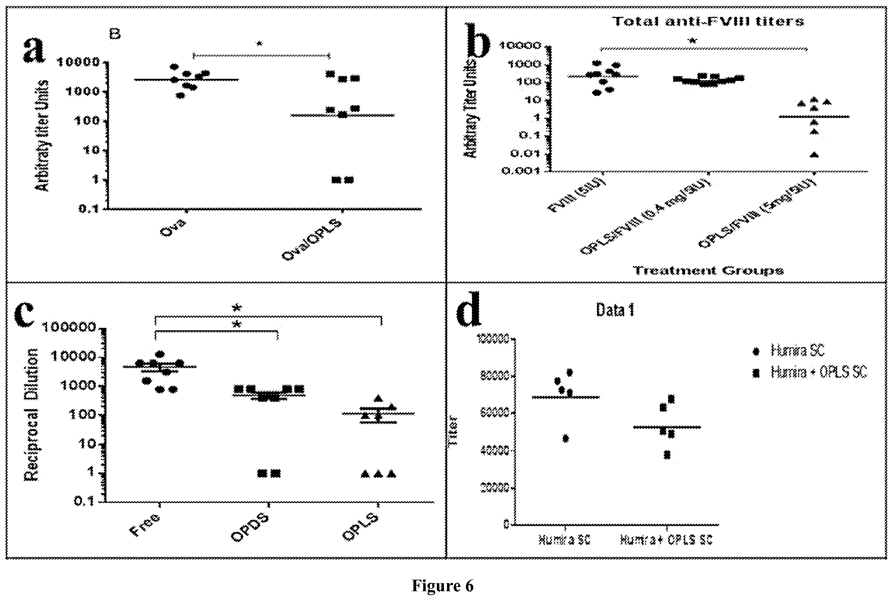

As shown in Figure 6(a), anti-Ova antibody titers were lower in mice that were co-administered OPLS (Sigma) (5 mg dose) and Ova (Sigma) (1 ug) via the s.c. route than in those administered Ova (1 ug) alone (also via the s.c. route). In this case, the lack of binding between OPLS and Ova suggest that the reduction in immunogenicity seen when OPLS is co-administered with Ova is due to the above-described immunological mechanism (distinct from tolerance induction).

### EXAMPLE 3

### This example describes the effect of OPLS on the Immunogenicity of Acid Alpha Glucosidase Protein (GAA)

Materials and Methods: GAA Source: Full length recombinant rhGAA was produced in Human Kidney Embryo Cells for our use by Creative Biomart (Shirley, NY); OPLS Source: Sigma-Aldrich (St.Louis, MO). n=8 animals/ group were immunized with 4 weekly s.c. injections of 1 ug of GAA in 100 ul of either sterile TRIS for the free group or in 5mg OPLS in sterile TRIS for the OPLS-GAA group. A group of 8 mice will receive the same dose of CBrhGAA in 5mg O-phospho-D-serine (OPDS) solution as a negative control. After a 2 week rest period, a blood sample from the saphenous vein will be collected. Samples were stored at -80°C until analysis. These samples were then analyzed for anti-GAA Ab titers. Anti-GAA antibody titers were measured as follows: ELISA plate (maxisorp, NUNC) was coated with 50ul of 5ug/ml of sterile rhGAA in sodium carbonate-bicarbonate buffer pH = 9.6. The plate was incubated overnight at 4°C. The plate was washed six times with PBS containing 0.05% Tween 20 (PBS-T, 250ul/well) using an automated plate washer. The plate was blocked with PBS containing 1% BSA (300ul/well) as the block buffer for 2hr at 37°C. The plate was washed six times with PBS-T. A serial dilution of samples (100ul/well) were then added to the plate in duplicate. The samples incubated for 1 hr at 37°C. The plate was washed six times with PBS-T. Detection antibody (1:10000 dilution and 100ul/well) in block buffer was added to the wells and incubated for 1 hr at at room temperature. The plate was washed six times with PBS-T. 3,3',5,5'-Tetramethylbenzidine substrate (100ul/well) was added to the wells and incubated for 30min at room temperature. The reaction was stopped with 2 N sulfuric acid and read immediately at 450 nm using a micro-plate reader. The titer values are quantitated using a statistical endpoint method as described by Frey et al. Control plasma samples were run on each plate. Statistical Analysis: Statistical difference (p<0.05) one-way ANOVA followed by Dunnette's post-hoc multiple comparison test using GraphPad Prism (Frey et al., J. Immunol. Methods, 1998. 221(1-2): p. 35-41).

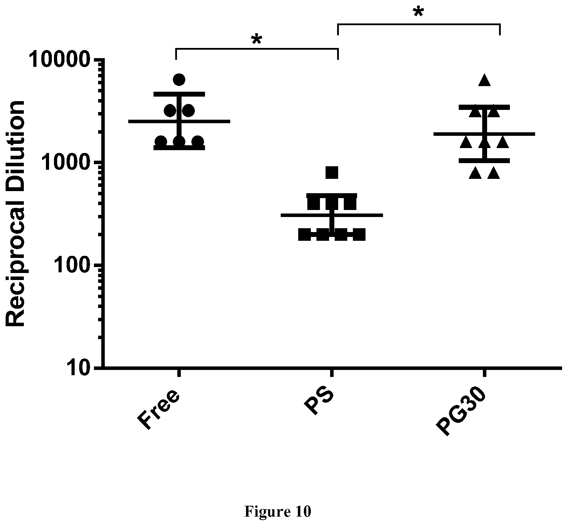

As shown in Figure 6(c), anti-GAA antibody titers were lower in mice that were co-administered either: (a) OPLS (5 mg dose) and GAA via the s.c. route, or (b) OPDS (5 mg dose) and GAA via the s.c. route, than in those administered GAA alone (also via the s.c. route). Anti-GAA titer levels were lowest in mice co-administered OPLS and GAA. The slight reduction in anti-GAA antibody titers seen in mice co-administered OPDS and GAA is likely attributable to conversion of OPLS to OPDS *in vivo.* In this case, the lack of binding between OPLS and GAA suggest that the reduction in immunogenicity seen when OPLS is co-administered with GAA is due to the above-described immunological mechanism (distinct from tolerance induction). Similar findings were observed following co-administration of PS-Liposomes with GAA (Figure 10).

### EXAMPLE 4

### This example describes the effect of OPLS on the immunogenicity of Adalimumab.

Methods: n=8 animals/ group were immunized with 4 weekly sc injections of 10 ug of adalimumab in 100 ul of either sterile TRIS for the free group or in 5mg OPLS in sterile TRIS for the OPLS-Adalimumab group. After a 2 week rest period, a blood sample from the saphenous vein was collected for titer analysis. Titer values were obtained by classical sandwitch ELISA. Briefly, Nunc-Maxisorb 96-well plates were coated overnight at 4 °C with 50 µl/well of 2.5 µg/ml Humira. Plates were then washed and blocked with 1% bovine serum albumin (blocking buffer). 50 µl/well of various dilutions of the sample were added to the plate and incubated at room temperature for 2 h. Plasma samples from animals exposes to a sham treatment (buffer only) served as controls. The plates were then washed and incubated with 50 µl of goat anti-mouse anti-body-HRP conjugate at a 1:50000 dilution in blocking buffer at room temperature for 1 h. Color was developed for 20 min with 100 µl of TMB solution. 100 ul of 2 N H₂SO₄ was used to stop the reaction. Optical density at 405 nm was measured using a plate reader. Non-responders were given the value of the plate specific factor for uniformity.

As shown in Figure 6(d), anti-Humira antibody titers were lower in mice that were co-administered OPLS (5 mg dose) and Humira® (10 mg/g) via the s.c. route than in those administered Humira® alone (10 mg/g) (also via the s.c. route) after 4 weekly injection followed by a 2 weeks washout period. Titer values were obtained by classical sandwitch ELISA were the plates was coated with Humira before incubation with dilutions with plasma samples, anit-Humira antibodies were detected with a rat anti-mouse-HRP conjugated antibodies. In this case, the weak binding between OPLS and Humira® suggest that the reduction in immunogenicity seen when OPLS is co-administered with Humira® is due to the above-described immunological mechanism (distinct from tolerance induction). It is highly doubtful that the weak binding seen here is "shielding" an immunologic epitope.

### EXAMPLE 5

### This example describes the effect of OPLS on Human IgG Immunogenicity.

Materials and Methods: 8 animals were used for this pilot study, 4 animals were injected with Human-IgG (Sigma) alone (1ug) or with Human IgG plus OPLS (1ug human IgG plus 0.4mg OPLS). The study was done with 4 injections over 2 weeks period as follows: day 1, day 8, day 10 and day 12 followed by a 3 days washout period. ELISA was done to measure total titers. Briefly, Nunc-Maxisorb 96-well plates were coated overnight at 4 °C with 50 µl/well of 2.5 µg/ml Human IgG. Plates were then washed and blocked with 1% bovine serum albumin (blocking buffer). 50 µl/well of various dilutions of the sample were added to the plate and incubated at room temperature for 2 h. Plasma samples from animals exposes to a sham treatment (buffer only) served as controls. The plates were then washed and incubated with 50 µl of goat anti-mouse anti-body-HRP conjugate at a 1:50000 dilution in blocking buffer at room temperature for 1 h. Color was developed for 20 min with 100 µl of TMB solution. 100 ul of 2 N H₂SO₄ was used to stop the reaction. Optical density at 405 nm was measured using a plate reader. Non-responders were given the value of the plate specific factor for uniformity.

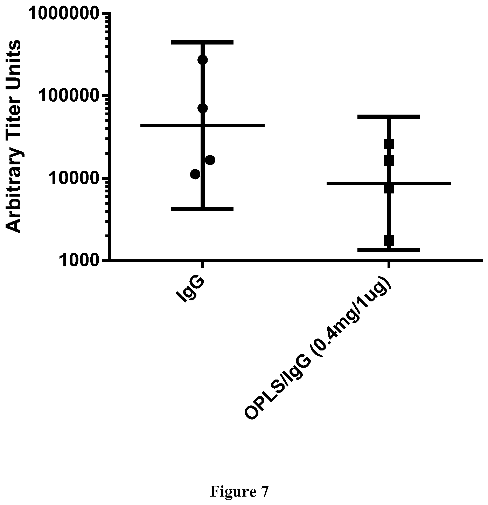

In this pilot study, animals were injected with Human-IgG (Sigma) alone (1ug) or with OPLS (5mg). The study was done with 4 injections over 2 weeks period as follows: day 1, day 8, day 10 and day 12 followed by a 3 days washout period. Titers were obtained by classical ELISA as mentioned above. As shown in Figure 7, anti-IgG antibody titers were lower in mice that were co-administered OPLS (0.4 mg dose) and IgG via the s.c. route than in those administered IgG alone (also via the s.c. route). It is expected that, as seen with other biotherapeutics tested, higher doses (5 mg or more) of OPLS will have an even more pronounced effect on titer levels when co-administered with IgG.

### EXAMPLE 6

### This example describes the effect of OPLS on Rituximab Immunogenicity (RXT).

Materials and Methods: 30 SW mice between 6 and 8 weeks of age were divided into 3 groups: group one received 1 ug RXT SC in the abdominal region. Group two received 1ug RXT/0.4 mg OPLS SC in the abdominal region and group 3 received 1ug RXT/5mg OPLS sc in the abdominal region. This treatment was given one day 1, 8, 10 and 12. Three days after the last injection the animals were sacrificed. Blood was centrifuged at 7500 rpm for 5 min at 4 degrees Celsius. Plasma was then removed and stored at -80 degrees Celsius until analysis Total titers were determined by classical standard ELISA. Briefly, Nunc-Maxisorb 96-well plates were coated overnight at 4 °C with 50 µl/well of 2.5 µg/ml RXT. Plates were then washed and blocked with 1% bovine serum albumin (blocking buffer). 50 µl/well of various dilutions of the sample were added to the plate and incubated at room temperature for 2 h. Plasma samples from animals exposes to a sham treatment (buffer only) served as controls. The plates were then washed and incubated with 50 µl of goat anti-mouse FC-specific anti-body-HRP conjugate at a 1:50000 dilution in blocking buffer at room temperature for 1 h. Color was developed for 20 min with 100 µl of TMB solution. 100 ul of 2 N H₂SO₄ was used to stop the reaction. Optical density at 405 nm was measured using a plate reader. Non-responders were given the value of the plate specific factor for uniformity. Statistics was done on the arithmetic mean of the log transformed data, graphs are reported as geometric means. Non-responders were given the value of the plate specific factor for uniformity.

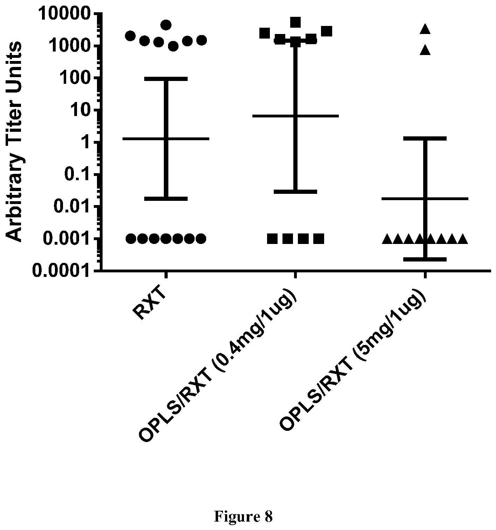

As shown in Figure 8, anti-RXT antibody titers were lower in mice that were co-administered OPLS in a dose dependent manner 5 mg vs 0.4 mg and RXT (1ug) via the s.c. route than in those administered RXT (1 ug) alone (also via the s.c. route).

### EXAMPLE 7

### This example describes the effect of OPLS and PS-Liposomes on Acid Alpha Glucosidase (GAA) Peptide Immunogenicity

Materials and Methods. GAA peptide Source: This GAA peptide was produced by American Peptide Company (Sunnyvale, CA). They produced for us a peptide 13 amino acids long containing amino acids 339-352 of the full length GAA sequence, YIFLGPEPKSVVQ. OPLS Source: Sigma-Aldrich (St.Louis, MO) Peptide concentration dose: 10 ug/ injection. Free GAA peptide (n=4), PS-GAA peptide (n=4), and OPLS-GAA peptide (n=5). Each mouse received 4 weekly subcutaneous (s.c.) injections near the base of the tail of 100 µl/injection of their treatment. Two weeks after the last injection, a retro-orbital blood sample was taken from each mouse. Samples were stored at -80°C until analysis. These samples were then analyzed for anti-GAA peptide Ab titers. Anti-GAA antibody titers were measured as follows: ELISA plate (maxisorp, NUNC) was coated with 100ul of 10ug/ml of sterile rhGAA in sodium carbonate-bicarbonate buffer pH = 9.6. The plate was incubated overnight at 40C. The plate was washed six times with PBS containing 0.05% Tween 20 (PBS-T, 250ul/well) using an automated plate washer. The plate was blocked with PBS containing 1% BSA (300ul/well) as the block buffer for 2hr at 37°C. The plate was washed six times with PBS-T. A serial dilution of samples (100ul/well) were then added to the plate in duplicate. The samples incubated for 1 hr at 370C. The plate was washed six times with PBS-T. Detection antibody (1:10000 dilution and 100ul/well) in block buffer was added to the wells and incubated for 1 hr at at room temperature. The plate was washed six times with PBS-T. 3,3',5,5'-Tetramethylbenzidine substrate (100ul/well) was added to the wells and incubated for 30min at room temperature. The reaction was stopped with 2 N sulfuric acid and read immediately at 450 nm using a micro-plate reader. The titer values are quantitated using a statistical endpoint method as described by Frey et al. Control plasma samples were run on each plate. Statistical Analysis: Statistical difference (p<0.05) one-way ANOVA followed by Dunnette's post-hoc multiple comparison test using GraphPad Prism.

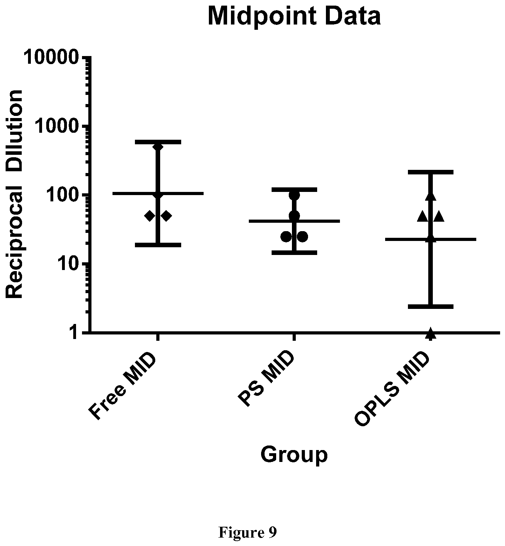

As shown in Figure 9, anti-GAA peptide antibody titers were lower in mice that were co-administered either: (a) OPLS (5 mg dose) and GAA peptide via the s.c. route, or (b) PS-liposomes (PS/PC,1:10,000 protein:lipid ratio based on M.W. of protein) and GAA peptide via the s.c. route, than in those administered GAA peptide alone (also via the s.c. route). Anti-GAA peptide titer levels were lowest in mice co-administered OPLS and GAA.

The GAA peptide did not bind to OPLS. Since the GAA peptide does not have internal fluorescence we needed to use a different method compared to the other protein-OPLS binding studies. Instead of using fluorescence to detect binding, ultraviolet/visible spectroscopy was used.

### EXAMPLE 8

### This example describes tolerance Induction towards GAA Protein using OPLS and PS-Liposomes.

Materials: Full length recombinant rhGAA was produced in Human Kidney Embryo Cells for our use by Creative Biomart (Shirley, NY). Dimyristoyl phosphatidylcholine (DMPC), phosphatidylglycerol (PG) and brain phosphatidylserine (PS) were purchased from Avanti Polar Lipids (Alabaster, AL .) O-phospho-L-serine (OPLS), Goat-anti-mouse IgG horseradish peroxidase, 3,3',5,5'-Tetramethylbenzidine (TMB) substrate were purchased from Sigma-Aldrich (St. Louis, MO). Animals: Breeding pairs of Pompe disease mice were purchased from a Jackson Laboratory (Bar Harbor, Maine). A colony homozygous for the knockout gene was established and animals aged from 8-12 weeks were used for the in vivo studies.

GAA Tolerance with OPLS. Methods: n=8 animals/ group were immunized with 4 weekly sc injections of 1 ug of GAA in 100 ul of either sterile TRIS for the free group or in 5mg OPLS in sterile TRIS for the OPLS-GAA group. A group of 8 mice will receive the same dose of GAA in 5mg O-phospho-D-serine (OPDS) solution as a negative control. After a 2 week rest period, a blood sample from the saphenous vein was collected. The mice were then re-challenged with 1 ug of free GAA in Sterile TRIS for 4 weeks. The mice were sacrificed after another 2 week rest period and plasma was collected than stored at -80C until analysis by ELISA for anti-GAA Ab Titers.

GAA Tolerance with PS-liposomes. Methods: n=8 animals/ group were immunized with 4 weekly sc injections of 1 ug of GAA in 100 ul of either sterile TRIS for the free group or PS liposomal formulation for PS group (30% PC, 70% DMPC). A group of 8 mice received the same dose of GAA in PG30 liposomal formulation(30% PG, 70% DMPC) as a negative, charge-matched control. After a 2 week rest period, a blood sample from the saphenous vein was collected. The mice were then re-challenged with 1 ug of free GAA in Sterile TRIS for 4 weeks. The mice were sacrificed after another 2 week rest period and plasma was collected than stored at -80C until analysis by ELISA for anti-GAA Ab titers.

Anti-GAA antibody titers. ELISA plate (maxisorp, NUNC) was coated with 50ul of 5ug/ml of sterile GAA in sodium carbonate-bicarbonate buffer pH = 9.6. The plate was incubated overnight at 4°C. The plate was washed six times with PBS containing 0.05% Tween 20 (PBS-T, 250ul/well) using an automated plate washer. The plate was blocked with PBS containing 1% BSA (300ul/well) as the block buffer for 2hr at 37°C.. The plate was washed six times with PBS-T. A serial dilution of samples (100ul/well) were then added to the plate in duplicate. The samples incubated for 1 hr at 37°C. The plate was washed six times with PBS-T. Detection antibody (1:10000 dilution and 100ul/well) in block buffer was added to the wells and incubated for 1 hr at at room temperature. The plate was washed six times with PBS-T. TMB substrate (100ul/well) was added to the wells and incubated for 30min at room temperature. The reaction was stopped with 2 N sulfuric acid and read immediately at 450 nm using a micro-plate reader. The titer values are quantitated using a statistical endpoint method as described by Frey et al. (Journal of Immunological Methods, 1998. 221(1-2): p. 35-41). Control plasma samples were run on each plate. Statistical Analysis: Statistical difference (p<0.05) one-way ANOVA followed by Dunnette's post-hoc multiple comparison test using GraphPad Prism.

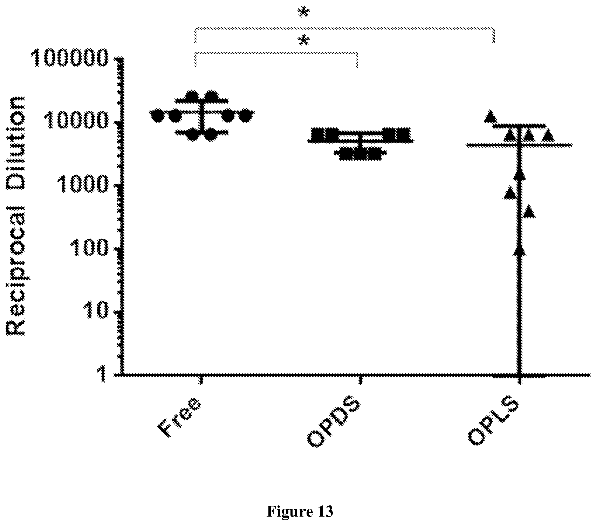

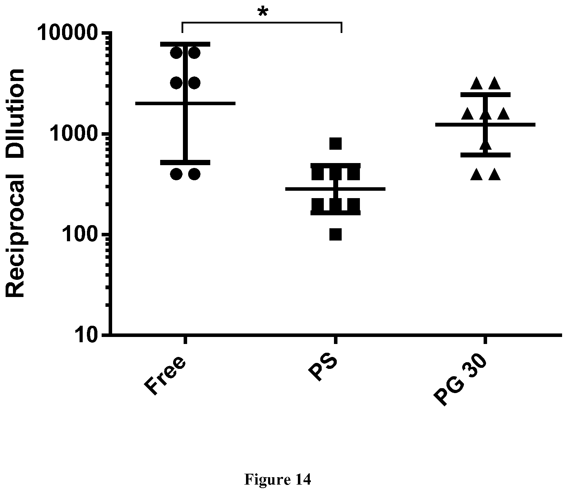

Results: The anti-GAA antibody titers after a GAA rechallenge were found to be significantly lower for the animals treated with OPLS-GAA compared to those treated with free GAA as seen in Figure 13. The anti-GAA antibody titers after a GAA rechallenge were found to be significantly lower for the animals treated with GAA-PS Liposomes compared to those treated with free GAA as seen in Figure 14.

### EXAMPLE 9

### This example describes Tregs in PS mediated hypo-responsiveness

Methodology: Immunization studies were conducted in hemophilia A mice model. The animals (n=3/treatment group) received S.C. injections of either free FVIII or FVIII-PS (2µg of FVIII) every week for 12 weeks. Two weeks after the last injection, the CD4+ T cells were isolated from spleen of the immunized animal and were stained with FITC conjugated to CD4 antibody and PE conjugated to anti FoxP3 antibody. The double positive cells were analyzed using flow cytometry. In order to account for the spectral overlap of FITC and PE, compensation using singly labeled FITC and PE controls were acquired and compensation was carried out using FlowJO software. Statistical analysis was carried out using 1-tail paired t-test using Minitab software.

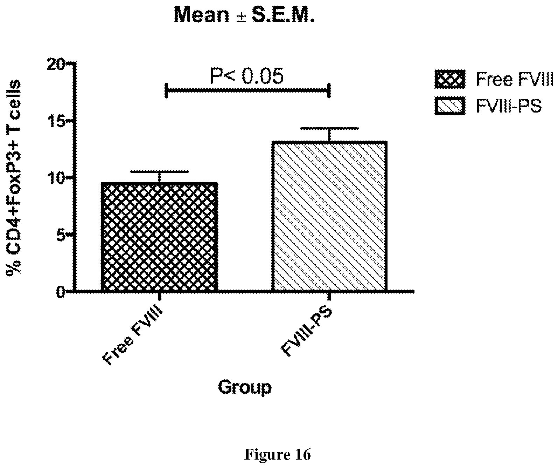

Results: The FoxP3 expression was analyzed as a biomarker for Treg. The CD4+FoxP3+ double positive cells were significantly higher for FVIII-PS treatment group compared to FVIII alone treatment group (Figure 16).

### EXAMPLE 10

### This example describes the role of Memory B cells in PS-mediated hyporesponsiveness.

Methodology: Immunization studies were conducted in GFP-FoxP3 knock-in hemophilia A mice model. The animals (n=7 or 8/treatment group) received S.C. injections of either free FVIII or FVIII-PS or FVIII-PG30 (2µg of FVIII) every week for 12 weeks. One group of animals (n=5) was left untreated and served as the naive control. Two weeks after the last injection, all animals were sacrificed and the memory B cells isolated from their spleens. Approximately 0.1*10^6 memory B cells from all mice were adoptively transferred via penile vein to a corresponding individual naive GFP-FoxP3 hemophilic mice. After a 48 h wait period, all the recipient animals were immunized aggressively with four weekly s.c. injections of 1 µg of free FVIII/ injection. Two weeks after the last injection, all animals were sacrificed and their plasma samples collected for analysis of antibody titer levels.

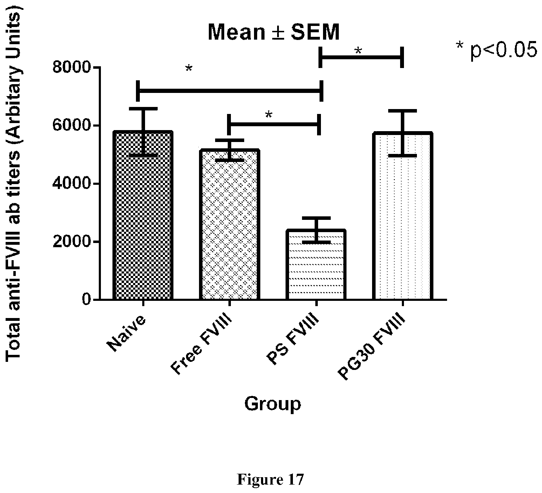

Results: The plasma samples were analyzed for the presence of total anti-FVIII antibody titer levels. Mice that received memory B cell from a FVIII treated or from a naive (untreated) mice generated robust FVIII immune response. In comparison, mice that received memory B cell from a PS-FVIII treated donor mice exhibited significantly reduced anti-FVIII antibody titers. However, memory B cells transferred from mice treated with FVIII associated with another negative charge PG lipid (FVIII-PG) failed to significantly reduce the antibody titers (Figure 17).

### EXAMPLE 11

### This example describes the requirement for proximity for inducing immune tolerance.

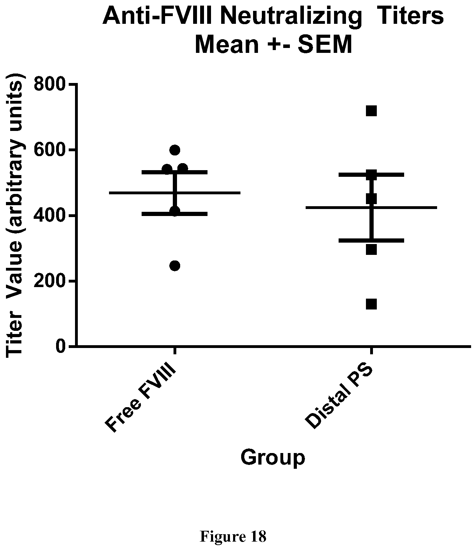

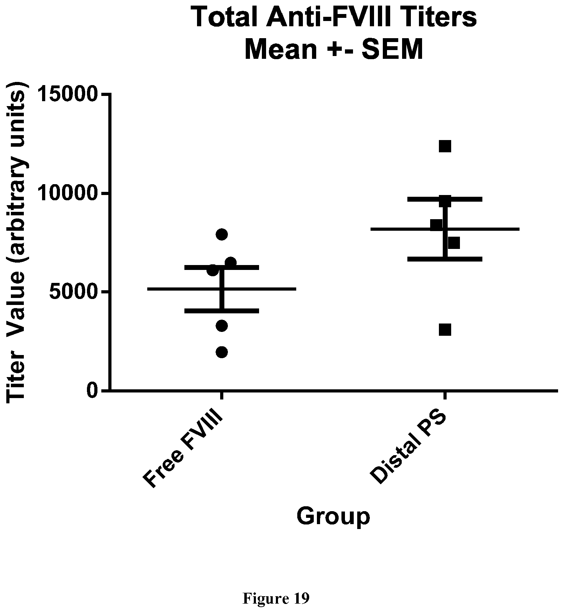

Mice were injected with unloaded PS liposomes in one flank and free FVIII in the other flank for four weeks, and then re-challenged with free FVIII for four more weeks before sacrifice. If spatial proximity is not critical, then the treatment with PS and FVIII spatially separated should yield a reduction in titers. However, the data showed that both neutralizing (425±100 SEM, n=5) and total anti-FVIII (8197±1235 SEM, n=5) titers were not significantly different than free FVIII control, which had neutralizing titers of 469±63.3 SEM (n=5), and total anti-FVIII titers of 5153±893 SEM (n=5) (Figs. 18 and 19, respectively). No significant difference was seen between the two treatment groups. This suggests that spatial proximity is required for the induction of an enduring hyporesponsiveness to FVIII by the PS formulation.

The development of an immune response against exogenous FVIII administration is of major clinical concern. Neutralizing antibodies develop in 15-30% of patients undergoing replacement therapy, reducing the efficacy of this treatment for patients with hemophilia A. Current strategies to reverse this immunogenicity require the administration of large doses of FVIII with immunosuppressants for an extended period of time, frequently up to one year. This strategy is both costly and carries with it considerable risk for the patient who will have their immune system compromised during therapy. Thus, developing new ways to reduce the immunogenicity to FVIII is extremely important.

Previous studies have shown that the complexation of FVIII with PS reduces the immune response against FVIII in hemophiliac mice. The induction of hyporesponsiveness to FVIII by the PS-FVIII formulation can be thought of as a 'reverse vaccination' strategy. Rather than attempting to establish an immune response against an antigen, the goal of therapy is to desensitize the patient against an antigen. If the mechanism in which this is achieved resembles the way in which apoptotic cells induce tolerance to their intracellular proteins, then this hyporesponsiveness should be antigen specific. A nonspecific response would also be clinically undesirable, as it would lead to immunosuppression.

In order for this hyporesponsiveness to develop, spatial proximity should be required between FVIII and PS, as apoptosis is a local event which occurs on the cellular level. To test this, mice received unloaded PS liposomes in one flank and free FVIII in the opposite flank, and were re-challenged with free FVIII. These mice showed no difference in immune response to FVIII when compared to free FVIII control. This data suggests that close anatomical proximity is required in order for hyporesponsiveness to develop. This observation lends support to the theory that PS-FVIII uses apoptotic mimicry to induce tolerance to FVIII.

In conclusion, it is clear from this data that the immunologic effects of PS-FVIII liposome formulations are specific to both FVIII as an antigen as well as the anatomical proximity of FVIII to the PS containing liposomes. These conclusions are consistent with the hypothesis that tolerance induction via apoptotic mimicry is the mechanism for the observed reduction in immune response as a result of reverse vaccination PS-FVIII therapy. Future studies should address the possibility of PS containing liposome formulations to reverse established titers, as well as induce hyporesponsiveness to an antigen other than FVIII.

### EXAMPLE 12

### This example describes apoptotic Induction In Dendritic Cells

Phosphatidylserine is a naturally occurring lipid and is part of vesicles released by apoptotic and other cell types and includes exosomes, ectosomes, microvesicles, membrane particles and apoptotic bodies. In one embodiment, proteins could be loaded into these particles after isolation and could be used to reduce immunogenicity and/or induction of tolerance. Dendritic cells were isolated from bone marrow progenitors of factor VIII deficient mice. Briefly, bone marrow cells were collected from femur and tibia. The cells were cultured for 8 days with change of media every two days. On day 8, cells from the suspension RPMI medium were collected, centrifuged, and plated in fresh plates. Immature dendritic cells were then used for apoptotic induction.

Apoptotic induction in dendritic cells was tested in several ways. Some of the methods tested were UV irradiation using 8W UV transilluminator for 1hr/5mins time points, by adding 5mM sodium butyrate total concentration, heat shock at 45°C for 1hr, and serum depletion for 24hrs. Cells were pelleted down after 48hrs of induction at 300g for 10mins to remove cell debris followed by 2000 - 10,000g for 20mins and 100,000g is required for exosomes. The apoptotic induction efficiency was later tested using Annexin V/Propidium Iodide kit from Sigma using fluorescent microscope. These apoptotic bodies and veiscles can be loaded with proteins (FVIII, GAA, Humira etc) to reduce immunogenicity and induction of tolerance.

### EXAMPLE 13

### This example describes characterization of safety of OPLS

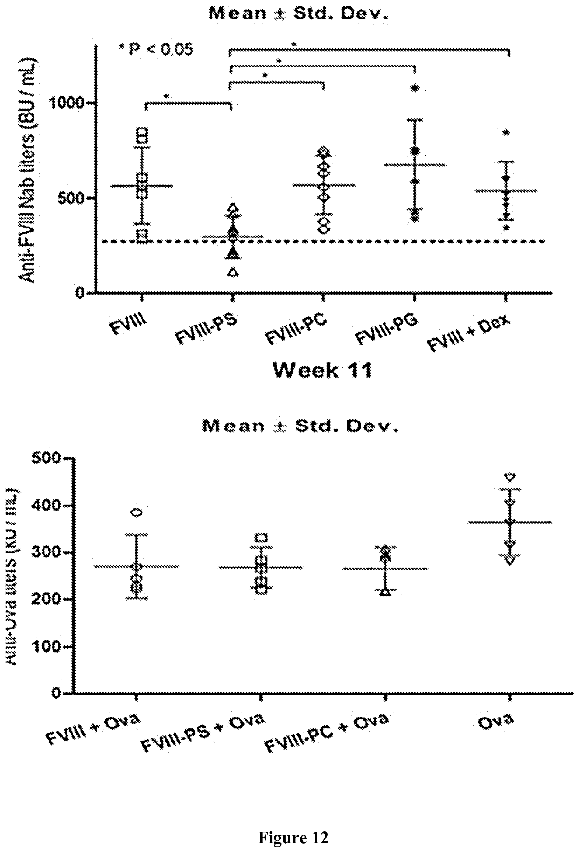

Given that the methods disclosed herein appear to work best with doses of OPLS that equal or exceed 5 mg, it is important to understand the safety and toxicity of high doses of OPLS. As shown in Figures 11-14, repeated, high (up to 950 mg) doses of OPLS are well tolerated in both rodent and primate models.

### EXAMPLE 14

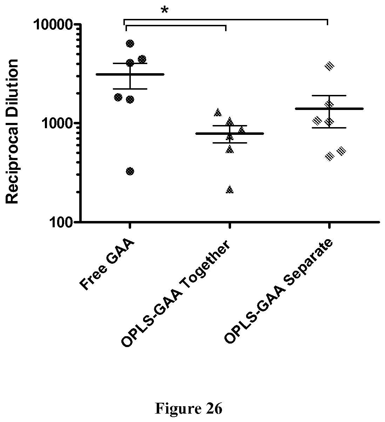

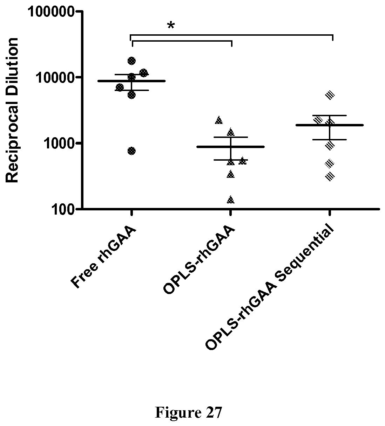

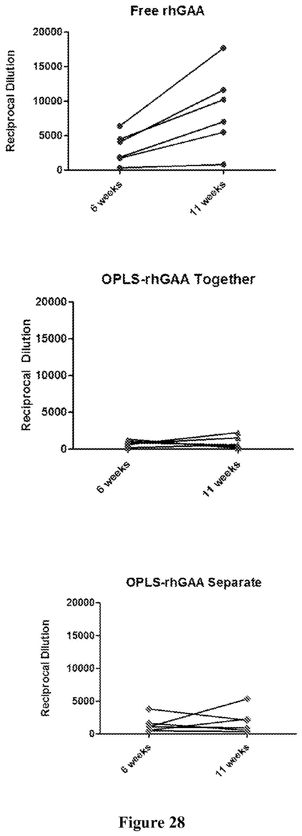

This examples describes OPLS-GAA sequential administration study. To determine if OPLS can exert its immunomodulatory effects on the immune response to GAA when the two are administered in separate spaced injections, at the same injection site the following experiments were carried out. Mice (n=6 animals/ group) were immunized with 4 weekly sc injections as follows. One group of animals received one single combined injection of 5mg OPLS and 1 ug rhGAA(100 ul volume). Recombinant human GAA was produced by Creative Biomart. The second group received one injection of 5 mg OPLS (100 ul volume) then after a 5 minute wait they received a second injection of 1 ug rhGAA in sterile TRIS (100 ul volume) in the same injection site. The final control group received one injection of sterile TRIS (100 ul volume) then after a 5 minute wait they received a second injection of 1 ug rhGAA in sterile TRIS (100 ul volume) in the same injection site. After a two week rest period a saphenous blood sample was collected. All of the mice were then rechallenged with 4 weekly injections of 1 ug of rhGAA in 100 ul sterile TRIS (one single injection). Analysis was done by ELISA for total titers. The results are shown in Figures 26-28. The mean free GAA after 6 weeks was 3135 ± 910, When co-administered, the GAA was 787 ± 155, and when administered separately (sequentially), the GAA was 926 ± 202 (Figure 26). After re-challenge, terminal sampling was done at week 11. The results are shown in Figure 27. The mean free GAA after 11 weeks was 8775 ± 2363, When co-administered, the GAA was 895 ± 335, and when administered separately (sequentially), the GAA was 1884 ± 761. This indicates that titers of mice that received either GAA co-administered with 5 mg OPLS or GAA administered into the same site 5 minutes after an injection of 5 mg OPLS are significantly lower after rechallenge compared to titers in animals that received free GAA. Either administration method was able to induce a hypo-responsiveness towards GAA. The combined results from 6 and 11 weeks are shown in Figure 28.

### EXAMPLE 15

In this example, we tested the effects of hypertonic buffers. We generated hypertonic buffer systems using three different excipients: NaCl, mannitol and O-Phospho-L-Serine (OPLS). NaCl was chosen as a generic salt to manipulate osmolarity. Mannitol is a common GRAS (Generally Regarded as Safe) excipient used in protein formulations. OPLS is the head group of the immune-modulatory lipid Phosphatidylserine (PS). All three excipients are highly soluble in aqueous media. We tested the effects of these buffers on rituximab pharmacokinetics after sc administration in Swiss Webster mice.

Material and Methods: Swiss Webster mice (19-22 g) (SW) were from Charles River Laboratories (Wilmington, MA). All animal experiments were conducted in accordance with guidelines established by Institutional Animal Use and Care Committee at the University at Buffalo, State University of New York.

Commercial preparation of rituximab (RXT) was used. Rat anti-rituximab antibody was purchased from AbD Serotec (Raleigh, NC). Goat anti-mouse FC-specific HRP conjugated antibody, 3,3'5,5' tetramethyl benzidine (TMB) substrate system, Bovine serum albumin (BSA), O-Phospho-L-Serine (OPLS) and mannitol were purchased from Sigma (St. Louis, MO). All other solvents and buffer salts were obtained from Fisher Scientific (Fairlawn, NJ) or from Sigma (St. Louis, MO).

Preparation and characterization of injection buffers: One isotonic and six different hypertonic TRIS buffers were prepared to investigate the effects of hypertonicity and buffer composition on rituximab lymphatic uptake and plasma exposure (Table 1.).

**Table 1**

| Buffer | Composition | | | | | |
|---|---|---|---|---|---|---|
| | Tris | NaCl | OPLS | mannitol | pH | Osmolarity |
| | mM | mM | mM | mM | | mmol/kg |
| A | 25 | 150 | - | - | 7.5 | 300 |
| B | 25 | 300 | - | - | 7.5 | 600 |
| C | 25 | 300 | 20 | - | 7.5 | 600 |
| D | 25 | 300 | - | 20 | 7.5 | 600 |
| E | 25 | 150 | 270 | - | 7.5 | 600 |
| F | 25 | 150 | - | 270 | 7.5 | 600 |

Isotonic TRIS buffer was prepared using 25 mM TRIS and 150 mM NaCl (Buffer A). Hypertonic (600 mmol/kg) TRIS buffers were prepared with 25 mM of TRIS containing 300 mM NaCl (Buffer B). Buffers "C" and "E" contained NaCl as well as 20 mM of OPLS or Mannitol. To further delineate the effects of buffer composition on lymphatic uptake, we prepared two buffers at 600 mmol/kg with a 270 mM of OPLS (Buffer D) or mannitol (Buffer F). Since osmolarity of these buffers is the same as buffers "C" and "E", any changes in lymphatic uptake will be attributed to increase in OPLS and mannitol concentrations. pH was adjusted to 7.5. Osmolarity was measured using a 5500 Vapor Pressure Osmometer (Wescore Inc. Logen UT, USA) according to manufacturer's instruction.

Rituximab pharmacokinetics studies: 126 male SW mice were divided into 7 groups. Each group consisted of 18 animals, three for each time point of the PK profile. Each animal was given 1ug/g RXT formulated in one of the formulations described above (table 1). All sc injections were in the abdominal region equidistance from the inguinal lymph node. Since absorption is expected to be complete by 5 day, the following preset time points 1, 5, 15, 24, 48, and 120 hr were chosen for sacrifice and sample collection. Total blood and both inguinal lymph nodes were collected. The disposition of RXT will be determined from the iv PK profile and convoluted with the absorption data generated from the sc studies. For iv PK study, animals were given 1ug/g RXT in base buffer (buffer A) via the penile vein. Total blood was collected at the following times points: 0.5, 2, 15, 24, 48, and 120 hr. Blood was centrifuged at 7500 rpm for 5 min at 4 degrees Celsius. All samples were stored at -80 degrees Celsius until analysis. Immediately before analysis; the inguinal lymph nodes were homogenized in 300 µl of PBS on ice and then analyzed.

Analysis of RXT in plasma and lymph node samples: Analysis of plasma and lymph node samples from PK studies was done with a standard ELISA. Briefly Nunc-Maxisorb 96-well plates were coated overnight at 4°C with 100 µl/well of 1 µg/ml anti-RXT antibody (AbD Serotec Raleigh, NC). Plates were then washed and blocked with 1% bovine serum albumin (blocking buffer). Samples were diluted as needed to a final dilution of 1:100 or 1:1000 in blocking buffer and added to the plate. A serial dilution of RXT in blocking buffer also added to the plate for the standard curve. 100 µl of goat anti-human-HRP conjugate anti-body at a 5ug/ml was used as a detection anti-body. Color was developed for 20 min with 100 µl of TMB solution. 50 ul of 2 N H2SO4 was used to stop the reaction. Optical density at 450 nm was measured using a plate reader.

Results: pharmacokinetics of rituximab in SW mice after iv and sc administration: The plasma concentration-time profile of rituximab after iv and sc administration of a 1ug/g dose in isotonic buffer A is shown in figure 29. Based on the data, the sc bioavailability of rituximab in Buffer A is 0.29 (29%). The fraction of the dose taken up by the lymph nodes is estimated at 0.05 (5% of the total absorbed dose).

Effect of hypertonic buffer composition on lymphatic uptake of rituximab: To elucidate the effects of hypertonic buffer on lymphatic uptake we compared the concentration of rituximab in excised inguinal lymph nodes after sc administration of 1ug/g of rituximab in isotonic buffer (Buffer A) and hypertonic buffer (Buffer B) in SW mice. As shown in figure 30, we observed an increase in lymph node concentration of rituximab administered in the hypertonic buffer B as compared to Buffer A. rituximab was recovered from the lymph nodes of animals in the first 2 time points in Buffer A group, the amount of rituximab in the lymph nodes of animals in the remaining time points was below the limit of detection of our ELISA. However, all animals in buffer B group had rituximab in their lymph node over the entire course of the study.

To further delineate the effect of hypertonic buffer composition on lymphatic uptake, we compared lymph-node concentration of rituximab administered in the remaining 5 buffers in table 1 to the results obtained from buffer B. As shown in Figure 31A there is no substantial increase in lymph node concentration of rituximab administered in 20 mM OPLS hypertonic buffer (buffer C) as compared to buffer B, however, 270 mM OPLS (buffer E) resulted in an increase in lymph node concentration as compared to buffers B and C. Since the osmolarity in all three buffers is the same (600 mOsmol) the resulting increase in the fraction of the dose taken up by the lymph nodes, and the resulting increase in lymph node concentration, could be attributed to increased OPLS concentration in buffer E vs B and C.

Interestingly, when mannitol was used (buffers D and F), we observed an increase in lymph node concentration of rituximab administered in either mannitol-containing buffers as compared to mannitol-free buffer B (figure 31B). Unlike OPLS, this increase was independent of manntiol dose. The model estimated the fraction of the dose taken up by the lymph nodes to be comparable in both buffers D and F; this was also comparable to the estimate obtained for the high dose OPLS (buffer E). This data further supports the notion that buffer composition plays a role in lymph node uptake since osmolarity was held constant in all three buffers.

Effect of hypertonic buffer composition on rituximab plasma concentrations and bioavailability: To investigate whether improved lymphatic uptake would translate to improved bioavailability of rituximab, plasma samples were analyzed to determine rituximab concentrations and then modeled as above. As shown in figure 29, we observed an increase in the plasma concentration of rituximab administered in the hypertonic buffer B as compared to the isotonic buffer A. Next we compared the effect of buffer composition on bioavailability and plasma exposer by comparing plasma concentration time profile of rituximab administered in the remaining 5 buffers to that of buffer B. Figure 5A shows the results obtained from OPLS-containing buffers (C and E). The data shows increased rituximab plasma concentration for both buffers as compared to buffer B especially at latter time points. Similarly, mannitol-containing buffers D and F resulted in higher rituximab plasma concentration as compared to buffer B.

Overall hypertonic buffers resulted in higher rituximab bioavailability as compared to the isotonic buffer. Furthermore, the buffer composition affected the bioava
Our data shows that lymphatic uptake was improved when rituximab was administered in hypertonic (600 mmol/Kg). Furthermore, the composition of the buffer played a role in lymphatic uptake, mannitol and OPLS performed better than osmolarity-matched buffers containing NaCl alone. The improved lymphatic uptake was closely correlated with improved plasma exposure and improved bioavailability of rituximab.

While not intending to be bound by any particular theory, it is considered that hypertonic buffers in the sc space alter the environment in the interstitial milieu. This environment is the driving force behind the formation of interstitial fluid. Under normal conditions the blood filters through the arteriolar end of the capillary bed, a process driven by the increased hydrostatic pressure at that end as compared to the venular end of the capillaries. Downstream of the capillaries, the oncotic pressure increase within the blood capillaries, favoring the re-absorption of the filtrate back into blood carrying with it waste and byproducts from the tissue. Hyper-osmolarity in the interstitium, resulting from hypertonic buffers alters the osmotic conditions in the interstitial milieu increasing the interstitial volume and resulting in increased lymphatic drainage. The effects of volume expansion by hypertonic buffers in the sc space can be expected to last until osmolarity is restored. This may explain the advantage of mannitol and high dose of OPLS over NaCl alone.

### EXAMPLE 16

### This example demonstrates that OPLS reduces immunogenicity by inducing tolerogenic properties in dendritic cells.

Materials and Methods: Full length recombinant human FVIII (Baxter Biosciences, Carlsbad, CA) was used. O-Phospho-L-Serine (OPLS) was purchased from Sigma (St. Louis, MO). All solvents and buffer salts were obtained from Fisher Scientific (Fairlawn, NJ) or from Sigma (St. Louis, MO). Anti-FVIII monoclonal antibody ESH8 was obtained from American Diagnostica Inc. (Greenwich, CT). Heat inactivated premium Bovine Serum Albumin (BSA) from Lonza Walkersville Inc. (Walkersville, MD). RPMI-1640 media was from Gibco Life Technologies (Carlsbad, CA). Endosafe Endochrome-K® kit was purchased from Charles River Laboratories, (Charleston, SC). NUNC MaxiSorp 96 well plates were from Thermoscientific (Waltham, MA). Cytokine ELISA kits were from R&D System Inc. (DuoSet) (Minneapolis, MN, USA) or from Invitrogen (Camarillo, CA). DilC18(5) was from Sigma (St. Louis, MO). BD FC-block, anti-CD40-PE, and anti-MHCII-FITC antibodies and respective isotypes were purchased from BD Biosciences (San Jose, CA) or eBiosciences (San Jose, CA)

Tolerance induction studies in HA mice: Breeding pairs of exon 16 knockout transgenic hemophilic mice (HA) were used. All animal studies were conducted in accordance with an approved protocol by the Institutional Animal Use and Care Committee at the University at Buffalo, State University of New York.

Immunization and re-challenge schedule: To delineate whether OPLS mediated reduction of antibody titer is due to immune tolerance or immune suppression, we performed re-challenge experiments. Forty HA mice were pre-treated with FVIII, FVIII plus OPLS or FVIII plus Dexamethasone once-a-week for four consecutive weeks by sc injections of endotoxin free formulation of 1µg of FVIII alone or with 5 mg OPLS or 200 ng Dexamethasone (Dex) as shown in figure 1. The injections were prepared in TRIS buffers containing 25 mM TRIS and 150 mM NaCl at pH 7.5. For OPLS containing formulations, OPLS was added to the buffer and the pH adjusted to 7.5. The appropriate amount of FVIII was added immediately before injection to prevent complexation between FVIII and OPLS pre-injection. Frequent administration of low dose of Dexamethasone (200 ng/injection) was preferred to avoid severe immune suppression of lymphocyte activity. This was followed by a 2-week washout period. Animals were then re-challenged aggressively with four weekly sc administrations of 1 µg of free FVIII. On the eleventh week, animals were sacrificed and blood was collected in 10 % acid citrate dextrose (ACD), centrifuged and plasma isolated. Samples were stored at -80°C until analysis. Total anti-FVIII titer levels were measured by ELISA.

Characterization of BMDC exposed to FVIII in the presence and absence of OPLS: In this set of experiments we examined the effects of OPLS on phenotypic maturation, cytokine profile and migratory potential of bone marrow derived dendritic cells (BMDC) exposed to FVIII in the presence and absence of OPLS.

Generation of bone marrow derived dendritic cells (BMDC): Bone marrow was isolated from 6 to 8 week old naive HA. Briefly, bone marrow was cultured in RPMI media supplemented with Fetal Bovine Albumin (low endotoxin), 2-mercaptoethanol, Pen/Strep and 200IU/ml GMCSF. Media was changed on days 3, 6 and 8 of culture and DCs were used on day 9. BMDC are then harvested and used as needed. To verify the differentiation of bone marrow cells into DCs, CD11c surface expression was confirmed by flow cytometry using anti-CD 11c-FITC antibody with appropriate isotope controls.

Effect of OPLS on phenotypic maturation of BMDC: Next we investigated the effects of OPLS on phenotypic maturation of BMDC. BMDCs were exposed to 1 ug/ml FVIII in the presence and absences of OPLS for 24 hr. The cells were then harvested, washed twice with ice cold sterile PBS and suspended at approximately 1 × 10⁶ cells/mL. Cells were blocked using BD Fc-block antibody before labeling with anti-CD40-PE or anti-MHCII-FITC antibodies. The cells were analyzed using a FACSCalibur flow cytometer (BD Biosciences, San Jose, CA). This study was conducted in triplicates.

Effects of OPLS on cytokine profile of BMDCs *in vitro*: The effects of OPLS on cytokine secretion by dendritic cells were investigated. On day 9 BMDCs, generated as above, were treated with FVIII as an antigen in the presence of increasing dose of OPLS. Changes in production of pro-inflammatory cytokines TNF-α and IL12p70 as well as the regulatory cytokine TGF-β were monitored as a function of dose. BMDCs were exposed to 1ug/ml FVIII with increasing dose of OPLS (0, 0.5, 1, 10, 25, 50 mM). 1 M stock of OPLS was prepared by adding the appropriate amount of OPLS directly to RPMI media, and pH was adjusted using sterile 3N and IN NaOH. Endotoxin free stocks were used (Endosafe Endochrome-K® kit Charles River Laboratories, Charleston, SC). Cells were plated in quadruplicates (n =4) and cultured for 48 hours. Media was collected at the end of the incubation period for cytokine analysis. TGF-β, TNF-α and IL-12p70 concentrations were determined using standard ELISA (R&D System Inc. Minneapolis, MN, USA or Invitrogen Amarillo, CA).

Effect of OPLS on migratory potential of BMDC: The effect of OPLS on *in-vivo* migratory potential of BMDCs treated *ex-vivo* with FVIII or FVIII/OPLS was investigated. On Day nine, 3x10⁶ cells were incubated overnight with 5ug FVIII or 5ug/50mM FVIII/OPLS. The next day, non-adherent cells were harvested and washed twice with ice-cold sterile PBS and were labeled with DilC(18)5 dye (Sigma, St. Louis, MO). Cells were incubated for 5 min at 37°C with DilC(18)5 at 2ug/ml (Sigma, St. Louis, MO). After 5 min, cells were washed twice with ice-cold sterile PBS. Incorporation of the dye was confirmed by imaging a sample of the cells with Ziess Axiovert 200M inverted microscope equipped with and AxiocamMRC colored camera and LEP motorized stage controller. Excitation was by Cy5 channel and emission filter was 695/655 nm. (Carl Zeiss Microscopy, LLC. Thornwood, NY). Nine HA mice were divided into 3 groups: Naive, FVIII and FVIII/OPLS. Total non-adherent cells recovered from above were divided equally between the three mice in each treatment group and were injected subcutaneously (sc) in the lower abdominal. 3 hr after injection the animals were sacrificed and the inguinal lymph nodes were removed and fixed in 10% paraformaldehyde for 24 hr. The lymph nodes were then imaged whole using a Bio-Rad ChemiDoc MP imaging system with an ImgeLab 4.0.1 software package (Bio-Rad Laboratories, Inc. Hercules, CA).

Immunogenic potential of BMDC exposed to FVIII and OPLS/FVIII: To ascertain the effects of OPLS on the ability of BMDC to initiate a robust immune response *in vivo*, we tested the immunogenic potential of BMDCs after *ex vivo* exposure to FVIII in the presence and absence of 50 mM OPLS. On day nine, BMDCs were treated with 5ug FVIII or 5ug/50mM FVIII-OPLS for 24 hr. Then, media was removed and cells were washed twice with ice cold sterile PBS. About 5x10⁵ cells/mouse were then injected sc into HA mice, in two groups, FVIII and FVIII-OPLS group. This was done once a week for 3 weeks followed by a 2-week washout period. At the end of the washout period animals were sacrificed and plasma was collected. Total anti-FVIII titer levels were measured by ELISA with the plate specific cutoff values calculated from plasma of naive animals.

Effect of OPLS on *in-vivo* immunogenicity of FVIII: 21 HA mice between 6 and 8 weeks of age were divided into 3 groups. Group one received 1 ug FVIII SC in the abdominal region. Groups two and three received 1ug FVIII with 0.4 mg OPLS/injection (group 2) or 5 mg OPLS /injection (group 3) sc in the abdominal region. This treatment was given on day 1, 8, 10 and 12. Three days after the last injection the animals were sacrificed. Blood collected and centrifuged at 7500 rpm for 5 min at 4 degrees Celsius. Plasma was then removed and stored at -80°C until analysis.

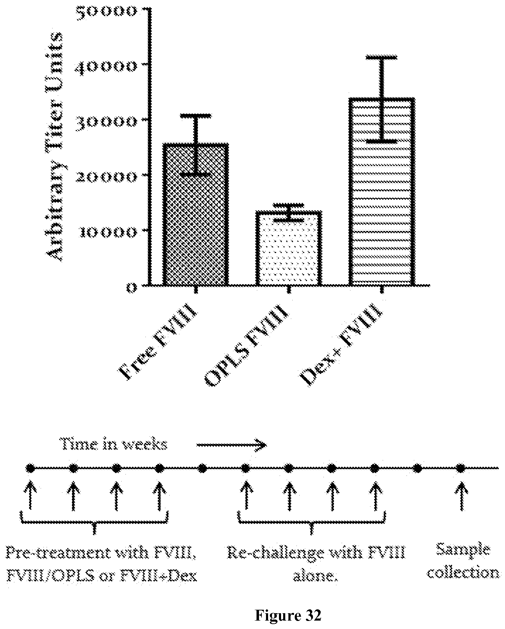

Statistics: Non-parametric one-way ANOVA with Tukey's post hoc analysis was used for the analysis of titer values where appropriate. Student t-test was used for comparing mean intensity of fluorescence (MIF) for flow cytomerty data. Non-parametric one way ANOVA with Kruskal-Wallis post hoc analysis was used for the analysis of titers values where appropriate
Results: OPLS induces hypo-responsiveness to FVIII re-challenge in HA mice: In order to investigate whether OPLS is the critical structural component responsible for induction of tolerance, a re-challenge study was conducted. During the pre-treatment stage both OPLS and Dexamethasone reduced anti-FVIII antibody response as expected. Upon re-challenge with FVIII, animals pre-treated with FVIII, and FVIII-Dexamethasone showed a robust anti-FVIII antibody response (25438 ± 5275 and 33694 ± 7598 inverse dilution factor respectively, mean ± SEM) as compared to animal pre-treated with FVIII-OPLS (14712 ± 1853 inverse dilution factor mean ± SEM, figure 32). Only OPLS pretreated group showed lower titers even after treatment with OPLS was stopped, but such an effect was not observed in the Dexamethasone treatment group.

OPLS mediated changes to BMDC maturation *in vitro*: The mechanism of antigen processing and presentation involves the following sequential steps; antigen uptake by dendritic cells is the first step in mounting an effective immune response. This is followed by phenotypic maturation, cytokine production and migration to afferent lymphatic for T-cell presentation. To determine whether OPLS interferes with antigen processing and presentation by DC, we monitored its effects on BMDC phenotypic maturation, cytokine secretion and migratory potential.

OPLS reduced the expression of co-stimulatory marker CD40 in BMDCs *in vitro*: Exposure of BMDC to FVIII resulted in an increase in CD40 expression. In the presence of 50 mM OPLS, the number of CD40 expressing BMDCs dropped from an average of 47.8% in FVIII treated cells to 26.5% in cells treated with FVIII-OPLS (n=3, p<0.05 figure 2A upper panel), this was also reflected in the mean fluorescence intensity (MFI) which dropped from average of 14.4x10³ (arbitrary fluorescent units) in FVIII treated cells to 9.45x10³ (arbitrary fluorescent units) in the presence of OPLS (n=3, p<0.05 figure 2B upper panel).

MHCII expression levels were not affected by exposure of BMDC to OPLS as seen in figure 33A and 33B (lower panels). The percent of MHCII expressing BMDC was 89.8% and 89.5% in the absence and presence of 50 mM OPLS respectively (n = 3). This was also reflected in the mean fluorescence intensity (MFI). Average MFI from the three experiments was 468x10³ and 449 x10³ (arbitrary fluorescent units) for FVIII treated cells and FVIII-OPLS treated cells. (n=3). This finding suggests that OPLS does not interfere with processing and presentation of FVIII via MHCII complex in BMDC.

OPLS induces a dose dependent decrease in pro-inflammatory cytokines and an increase in regulatory cytokine production by BMDCs: DC exposed to FVIII in the presence of OPLS showed a dose dependent decrease in the pro-inflammatory cytokines TNF-α and IL-12p70 (n = 4). At 50 mM a total shut down of IL-12p70 cytokine production and a substantial reduction in TNF-α (Figure 2C) was observed. This was accompanied by a dose dependent increase in the regulatory cytokine TGF-β (n = 4) (Figure 33D). This data suggests that BMDC primed with FVIII and 10 mM or higher of OPLS generates a cytokine profile similar to a tolerogenic DC (Figure 33C and 33D).

OPLS effects on migration potential of BMDCs: Next step in antigen uptake and processing involved migration of DC from tissue to afferent lymphatics. Tolerogenic DC are expected to have enhanced migration to the afferent lymphatic. We followed the migration of BMDC exposed *ex vivo* to FVIII or FVIII/OPLS in host HA mice by incorporating the fluorescent membrane label DilC18(5). This dye only fluoresces in the hydrophobic environment of the cell membrane, any detected fluorescence will be attributed to DilC(18)5/BMDC complex and not the free dye. Figure 2D and 2E shows the bright field images of BMDC's exposed to FVIII (E top panel) or FVIII/OPLS (F top panel), the lower panels show the same image taken by Cy5 channel (emission filter set at 695/655). The fluorescence observed in the membranes of BMDC is an indication of successful labeling with DilC(18)5. Upon sc injection of DilC18(5) labeled BMDCs cells into HA mice, we observed an increase in the amount of fluorescence recovered in the inguinal lymph node in mice injected with OPLS/FVIII treated BMDCs (as determined by the area under the fluorescent curve) compared to FVIII treated (45254 ± 4240 for OPLS/FVIII vs. 26709 ± 5018 for FVIII treated group)(Figure 33F). This increase is a direct reflection of the number of BMDCs that reached the lymphatic due to the migratory potential of OPLS primed dendritic cells.

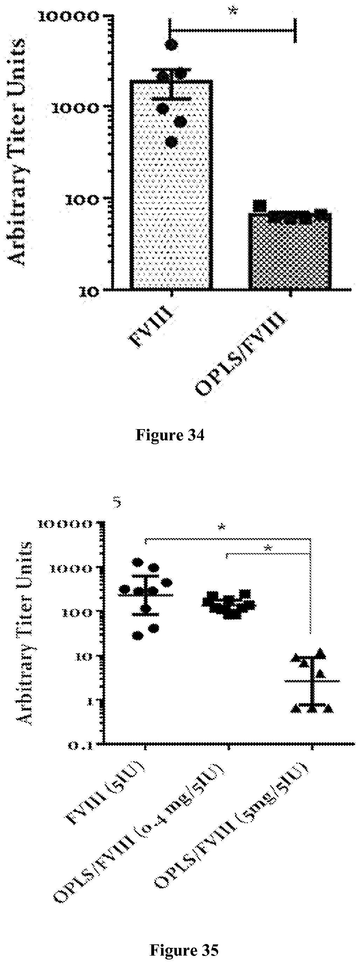

OPLS curtails the immunological potential of BMDC exposed *ex vivo* to FVIII in HA mice: In this study we investigated the effect of OPLS on BMDC's ability to initiate an immune response by monitoring antibody production in host HA mice after reintroduction of *ex vivo* treated BMDC. Our study shows statistically higher anti-FVIII titers in mice injected with BMDC treated *ex-vivo* with FVIII compared to those injected with FVIII/OPLS treated BMDC (1894 ± 667 vs. 81 ± 15.6 arbitrary titer units respectively) (mean ± SEM p = 0.03 Figure 34). It is important to note that mice in each group were not exposed directly to FVIII, rather to BMDCs that were exposed to FVIII or FVIII-OPLS *ex vivo.* Any response developed is the result of *ex vivo* matured BMDCs presenting FVIII antigen to the immune system of the host. This data indicate that OPLS impairs the ability of DC to present antigen in a manner conducive for an effective adaptive response.

Effect of OPLS on in-vivo immunogenicity of FVIII Figure 35 shows the results of *in vivo* immune response to FVIII in HA mice in the presence and absence of increasing dose of OPLS. Total anti-FVIII titer values in the FVIII treatment group were statistically higher than anti-FVIII titer in animals treated with high dose OPLS (285 ± 3.3 arbitrary units vs 5.34 ± 5.8 arbitrary units respectively) (geometric mean ± standard deviation about the geometric mean) (p<0.05). Titer values for the low dose OPLS treatment group were lower than FVIII alone, this reduction, however, was not statistically significant. (137 ± 1.4 vs. 285 ± 3.3 arbitrary titer units respectively).

This data clearly shows an *in vivo* dose dependent response to OPLS. In the present study OPLS was not complexed to FVIII. This allows for sequential administration by which OPLS is administered first to prime the immune system then the biologic is administered second.

Tolerance is induced by tolerogenic DCs. Tolerogenic dendritic cells are thought to be generated *in vivo* in response to an apoptotic event. They are phenotypically distinct from tissue resident immature DCs. They are characterized by increase in regulatory cytokine production and lower pro-inflammatory cytokine secretion. They show some degree of maturation as well as loss of adhesion molecules. The latter is required for migration out of the tissue into the afferent lymphatics. In the afferent lymphatics, these DCs can present antigens to T-cells in the absence of co-stimulatory markers resulting in T-cell non-responsiveness. Our data (figure 33) supports the formation of tolerogenic DC in response to OPLS. This is based on our observation of altered cytokine profile and down regulation of co-stimulatory marker CD40 while maintaining MHCII expression levels all of which are in agreement with tolerogenic DC characteristics.

Both immunogenic and tolerogenic DCs can migrate to the afferent lymphatics. In figure 2G we observed an increase in the amount of fluorescently labeled BMDC recovered in the inguinal lymph node of mice injected with BMDCs exposed to FVIII/OPLS as compared to FVIII alone. Upon reaching the lymphatics, DCs are expected to present the antigen via MHCI or MHCII to T-cells. The context of this presentation dictates the outcome of this interaction. Tolerogenic DCs can present the antigen to T-cells in the absence of co-stimulatory markers, such as CD40, resulting in tolerance. Tolerance should translate to reduced immunogenic potential *in vivo.* Data presented in figure 3 supports this notion. BMDCs exposed, *ex vivo*, to FVIII alone did not lose their immunogenic potential once transferred back into host HA mice. This was not the case for FVIII/OPLS treated BMDCs. This indicates that exposure to OPLS curtailed the immunological potential of BMDCs.

Data presented in this disclosure shows that the observed reduction in anti-FVIII titers in HA mice treated with OPLS/FVIII is the result of immunoregulation by OPLS and not immunosuppression. Furthermore, our data showed that, unlike Dexamethasone pretreated animals, OPLS pretreated animals were hyporesponsive to re-challenge with free FVIII. This not only indicates that OPLS is an immunemodulator but also indicates that the hyporesponsiveness to FVIII was antigen specific. This antigen specificity necessitates processing and presentation of FVIII by DCs in the context of lower CD40 expression accompanied by increased TGF-β production and reduced TNF-α and IL-12p70 production by DC in response to OPLS. This is further supported by our flow cytometry data showing no change in MHCII expression in response to OPLS. This mimics the properties of tolerogenic DCs. This is accompanied by an increase in the migratory potential of DC in response to OPLS and a curtailed immunogenic potential of DCs. Taken together our data indicates that OPLS has the critical structural properties required to alter the maturation of DC, leading to generation of DCs with tolerogenic properties.

The tolerogenic properties of OPLS can be utilized in a reverse vaccination strategy in which administration of low dose therapeutic protein with OPLS will desensitize the immune system of the patient to subsequent administration of the protein effectively reducing the immunogenicity of the therapeutic protein. We observed that this approach can be used to induce tolerance and reduced immunogenicity towards Acid Alpha-Glucosidase (GAA) in murine pompe disease models suggesting that this approach is generalizable. This reverse vaccination strategy with OPLS offers advantages over concomitant immune suppressive therapy such as dexamethasone, by offering antigen specific tolerance as opposed to general immune suppression.

While the disclosure has been described through specific embodiments, routine modifications will be apparent to those skilled in the art. Such modifications are intended to be within the scope of this disclosure.

## Claims

1. A composition for use in a method for inducing immune tolerance toward a protein antigen, wherein:
the composition is one of a first composition or a second composition, wherein the first composition comprises O-phospho-L-serine (OPLS), and the second composition comprises the protein antigen, wherein the first composition is free of the protein antigen and the second composition is free of OPLS; and
the method comprises administering the composition and the other of the first or second compositions separately through a subcutaneous route to an individual; wherein the two compositions are administered at anatomical sites that are less than 5.08 cm (two inches) apart from each other and within 60 minutes from each other.

2. The composition for use according to claim 1, wherein the first and the second compositions are administered concomitantly.

3. The composition for use according to claim 1, wherein the first and the second compositions are administered sequentially.

4. The composition for use according to claim 3, wherein the first composition is administered prior to the second composition or the first composition is administered after the second composition.

5. The composition for use according to claim 1, wherein OPLS is administered at a concentration of more than 20 mM.

6. The composition for use according to claim 5, wherein OPLS is administered at a concentration of from 20 to 300 mM.

7. The composition for use according to claim 1, wherein the osmolality of the first and the second composition is from 300 to 1,100 mmole/kg.

8. The composition for use according to claim 1, wherein the first and the second compositions are administered at least once a week for up to 12 weeks.

## Patentansprüche

1. Zusammensetzung zur Verwendung in einem Verfahren zur Induktion einer Immuntoleranz gegenüber einem Proteinantigen, wobei:
die Zusammensetzung eine von einer ersten Zusammensetzung oder einer zweiten Zusammensetzung ist, wobei die erste Zusammensetzung O-Phospho-L-Serin (OPLS) umfasst, und die zweite Zusammensetzung das Proteinantigen umfasst, wobei die erste Zusammensetzung frei von dem Proteinantigen ist und die zweite Zusammensetzung frei von OPLS ist; und
das Verfahren die Verabreichung der Zusammensetzung und die andere von den ersten oder zweiten Zusammensetzungen getrennt über eine subkutane Route an einen Patienten umfasst;
wobei die zwei Zusammensetzungen an anatomische Stellen wie folgt verabreicht werden: weniger als 5,08 cm (zwei Inches) entfernt voneinander und innerhalb 60 Minuten voneinander.

2. Zusammensetzung zur Verwendung nach Anspruch 1, wobei die ersten und die zweiten Zusammensetzungen gleichzeitig verabreicht werden.

3. Zusammensetzung zur Verwendung nach Anspruch 1, wobei die ersten und die zweiten Zusammensetzungen sequenziell verabreicht werden.

4. Zusammensetzung zur Verwendung nach Anspruch 3, wobei die erste Zusammensetzung vor der zweiten Zusammensetzung verabreicht wird oder die erste Zusammensetzung nach der zweiten Zusammensetzung verabreicht wird.

5. Zusammensetzung zur Verwendung nach Anspruch 1, wobei OPLS in einer Konzentration von mehr als 20 mM verabreicht wird.

6. Zusammensetzung zur Verwendung nach Anspruch 5, wobei OPLS in einer Konzentration von 20 mM bis 300 mM verabreicht wird.

7. Zusammensetzung zur Verwendung nach Anspruch 1, wobei die Osmolalität der ersten und der zweiten Zusammensetzung von 300 mmol/kg bis 1100 mmol/kg beträgt.

8. Zusammensetzung zur Verwendung nach Anspruch 1, wobei die ersten und die zweiten Zusammensetzungen mindestens einmal wöchentlich für bis zu 12 Wochen verabreicht werden.

## Revendications

1. Composition destinée à une utilisation dans une méthode d'induction d'une tolérance immunitaire vis-à-vis d'un antigène protéique,
la composition étant l'une d'une première composition ou d'une deuxième composition, la première composition comprenant de l'O-phospho-L-sérine (OPLS), et la deuxième composition comprenant l'antigène protéique, la première composition étant exempte de l'antigène protéique et la deuxième composition étant exempte d'OPLS ; et
la méthode comprenant l'administration de la composition et de l'autre de la première composition ou de la deuxième composition séparément par voie sous-cutanée à un sujet ;
les deux compositions étant administrées au niveau de sites anatomiques qui sont espacés de moins de 5,08 cm (deux pouces) l'un de l'autre et à moins de 60 minutes l'un de l'autre.

2. Composition destinée à une utilisation selon la revendication 1, dans laquelle la première composition et la deuxième composition sont administrées simultanément.

3. Composition destinée à une utilisation selon la revendication 1, dans laquelle la première composition et la deuxième composition sont administrées séquentiellement.

4. Composition destinée à une utilisation selon la revendication 3, la première composition étant administrée avant la deuxième composition ou la première composition étant administrée après la deuxième composition.

5. Composition destinée à une utilisation selon la revendication 1, l'OPLS étant administrée à une concentration supérieure à 20 mM.

6. Composition destinée à une utilisation selon la revendication 5, l'OPLS étant administrée à une concentration de 20 à 300 mM.

7. Composition destinée à une utilisation selon la revendication 1, l'osmolalité de la première composition et de la deuxième composition étant de 300 à 1 100 mmole/kg.

8. Composition destinée à une utilisation selon la revendication 1, la première composition et la deuxième composition étant administrées au moins une fois par semaine pendant 12 semaines au maximum.
